(19) Europäisches Patentamt
European Patent Office
Office européen des brevets

(11) Publication number : 0 474 601 A2

(12) EUROPEAN PATENT APPLICATION

(21) Application number : 91810690.7

(22) Date of filing : 29.08.91

(51) Int. Cl.$^5$ : **C12N 15/56,** C12N 15/29, C12N 15/63, C12N 1/21, A01N 63/00, // C12N15/70, C12N15/78

(30) Priority : 07.09.90 US 579457

(43) Date of publication of application : 11.03.92 Bulletin 92/11

(84) Designated Contracting States : AT BE CH DE DK ES FR GB GR IT LI LU NL SE

(71) Applicant : CIBA-GEIGY AG
Klybeckstrasse 141
CH-4002 Basel (CH)

(72) Inventor : Gaffney, Thomas D.
4607-G Hope Valley Road
Durham, NC 27707 (US)
Inventor : Lam, Stephen T.
8900 Jeanew Court
Raleigh, NC 27614 (US)

(54) **Expression of genes encoding plant pathogenesis related proteins in plant associated bacteria.**

(57) The present invention relates to a DNA sequence comprising regulatory components from a bacterial source fused to a DNA coding sequence from a plant, which DNA coding sequence results in expression of a plant pathogenesis related protein in a bacterium. It also relates to transgenic bacteria containing the DNA sequences and methods of using such transformed bacteria to provide plant protective activity in a localized environment.

EP 0 474 601 A2

This invention relates to expression of plant pathogenesis related proteins in bacteria and the use of such expression to provide antimicrobial activity or plant protective activity in the vicinity of the plant pathogenesis related protein expressing bacteria.

The beneficial effects of bacteria that naturally colonize the environment near plant roots (the rhizosphere) has been known for some time (Weller, 1988). One type of substance produced by such root colonizing bacteria that contribute to the antifungal benefits are hydrolases, particularly bacterial chitinases and bacterial glucanases and other glycosidase enzymes. Boller (1985) has reviewed the evidence that suggests that plant chitinases and β-1,3-glucanases are enzymes which inhibit plant pathogens. EP 171,381 discloses control of nematodes with soil bacteria (including pseudomonads), which express glycosidase enzymes.

Mauch et al. (1988a) describe that a variety of fungi, including plant pathogenic fungi, are inhibited by a combination of plant chitinase and β-1,3-glucanase proteins.

Some researchers have attempted to genetically engineer bacteria to express or secrete bacterial chitinases. US 4,751,081 discloses *Pseudomonas* bacteria expressing a heterologous chitinase gene. EP 290,123 discloses the use of *Streptomyces* chitinase signal sequence to secrete mature proteins from *Streptomyces*, and the use of this construction to control chitinase sensitive pests.

Roberts and Selitrennikoff (1988) compared plant endochitinases with bacterial exochitinases and concluded that only the plant enzymes have significant antifungal activity in vitro. Roberts and Selitrennikoff (1988) also noted that bacterial chitinases that are exo-chitinases are not as active against fungal targets as are the endochitinases of plant origin.

The present invention relates to a DNA sequence comprising:

a) regulatory components from a bacterial source fused to

b) a DNA coding sequence from a plant, which DNA coding sequence results in expression of a plant pathogenesis related protein in a bacterium.

Preferred DNA coding sequences are PR- 1A, PR- 1B, PR- 1C, PR-R major, PR-R minor, PR-P, PR-Q, PR-Q′, PR-2, PR-N, PR-O, PR-O′, SAR8.2a, SAR8.2b, cucumber chitinase/lysozyme, cucumber basic peroxidase, tobacco basic glucanase and tobacco basic chitinase.

Another aspect of the present invention is the bacteria that have been transformed with one or more of the DNA sequences of the invention.

Another aspect of the present invention is a method for providing plant protective activity in a localized environment comprising introducing into the localized environment one or more bacteria strains, wherein each of the bacteria strains contains a DNA sequence of the invention, said DNA sequence being capable of producing a plant protective amount of a pathogenesis related protein.

Another aspect of the present invention is a method comprising introducing into a localized environment a mixture of more than one bacterial strain, with the proviso that each bacterial strain in the mixture contains a different DNA sequence, said different DNA sequences being capable of producing different pathogenesis related proteins in the same localized environment resulting in an enhanced plant protective activity.

Another aspect of the present invention is a method for providing plant protective activity in a localized environment comprising introducing into the localized environment a bacterial strain containing more than one of the DNA sequences of the invention, each of said DNA sequences being capable of producing a plant protective effective amount of a pathogenesis related protein. A preferred localized environment is the rhizosphere.

Preferred bacteria are soil bacteria, particularly *Pseudomonas*.

To assist in interpreting the meaning and scope of the present invention, the following terms are intended to have the meanings as described below, unless otherwise indicated All references cited in this application are herein incorporated by reference for their relevant teachings.

Bacterial Strain: A race or subculture of a bacterial species exhibiting certain distinct phenotypic characteristics which enable it to be distinguished from other members of the same species.

Coding DNA Sequence: A DNA sequence which, when transcribed and translated, results in the cellular formation of a polypeptide.

Derived from: In the context of this application genes, parts of genes, or other DNA sequences "derived from" other DNA sources embraces genes, parts of genes or other DNA sequences, identical to or substantially homologous to the DNA source material.

Plant Protective Effective Amount: An amount of pathogenesis related protein sufficient to cause significant decrease in plant pathogenicity or significant inhibition of growth and division of certain microbial species or significant death or significant reduction of population of certain microbial species. Preferably, the microbial species are fungal species. Significant decrease in plant pathogenicity of certain microbial species means increased survival or health of plants exposed to the pathogensis related protein in the presence of such microbial species, as measured by, for example, decreases in number and size of lesions, increased emergence of seedlings, etc. Significant decrease in plant pathogenicity includes production by the pathogenesis related protein of a plant protective plant response. Significant inhibition of growth and division of certain microbial species or significant reduction of number of population of certain microbial species means inhibition of growth or division large enough to result in a net decrease in the number of members in the population of the certain species and eventually threaten the survival of the certain species in the localized environment and/or lead to eventual extinction of the species in the localized environment. Preferably, the net loss will be on the order of 50 % and more preferably the loss will be about an order of magnitude to about 10 % of the population in the absence of the pathogenesis related protein.

Gene: A discrete chromosomal or extrachromosomal (e.g., plasmid) region comprising regulatory DNA sequences responsible for the control of expression, i.e., transcription and translation, and of a coding sequence which is transcribed and translated to give a distinct polypeptide.

Hybrid Sequence or Hybrid Gene: A DNA sequence containing at least two heterologous parts, e.g., parts derived from, or having substantial sequence homology to, pre-existing DNA sequences which are not associated in their pre-existing states. The pre-existing DNA sequences may be of natural or synthetic origin.

Localized Environment: An environment that is spatially uninterrupted and that exhibits similar characteristics for microbial growth, e.g., exhibiting similar characteristics of nutrients, light, pressure, moisture, temperature, atmospheric content, chemical composition, etc. Such localized environments include the environment near the roots of plants both in the soil and submersed in water (hydroponic root environment).

Phenotypic Trait: An observable property resulting from the expression of one or more genes.

Plant cell: The structural and physiological unit of plants, consisting of a protoplast and the cell wall. The term "plant cell" refers to any cell which is either part of or derived from a plant. Some examples of cells encompassed by the present invention include differentiated cells that are part of a living plant; differentiated cells in culture; undifferentiated cells in culture; the cells of undifferentiated tissue such as callus or tumors; seeds; embryos; propagules and pollen.

Plant Tissue: A group of plant cells organized into a structural and functional unit. Any tissue of a plant in plants or in culture. This term includes, but is not limited to, whole plants, plant cells, plant organs, plant seeds, protoplasts, cell culture and any groups of plant cells organized into structural and/or functional units. The use of this term in conjunction with, or in the absence of, any specific type of plant tissue as listed above or otherwise embraced by this definition is not intended to be exclusive of any other type of plant tissue.

Plant Pathogenesis Related Protein: Any of a group of chemicals, preferably polypeptides, that are produced in plants that confer increased resistance to microbial cells, preferably increased resistance to fungal cells. Some of these compounds are commonly referred to as PR proteins. Preferred PR proteins are the hydrolases and glycosidases, particularly the chitinases and glucanases produced by plants. More preferably, PR proteins include PR-1A, PR-1B, PR-1C, PR-R major, PR-R minor, PR-P, PR-Q, PR-Q′, PR-2, PR-N, PR-O, PR-O′, SAR8.2a, SAR8.2b, cucumber chitinase/lysozyme, cucumber basic peroxidase, tobacco basic glucanase and tobacco basic chitinase.

Pre-existing DNA Sequence: A DNA sequence that exists prior to its use, in toto or in part, in a product or method according to the present invention. While such pre-existence typically reflects a natural origin, pre-existing sequences may be of synthetic or other origin.

Rhizosphere: An environment near the roots of plants both in the soil and submersed in water (hydroponic root environment). Near the roots means within a distance of the roots close enough that chemicals produced preferentially in the root cells are detectable.

Substantial Sequence Homology: Substantial functional and/or structural equivalence between sequences of nucleotides or amino acids. Functional and/or structural differences between sequences having substantial sequence homology will be de minimus. The sequences that differ from the natural sequences are usually variants of the natural sequence. A variant of a natural sequence is a modified form of the natural sequence that performs the same function. The variant may be a mutation, or may be a synthetic sequence. A de minimus functional difference results from a nucleotide or amino acid sequence that codes for a protein having essentially the same characteristics as the native protein. Such characteristics can include, for example, immunological reactivity, enzyme activity, structural protein integrity, etc. Structural differences are considered de minimus if there is a significant amount of sequence overlap or similarity between two or more different sequences or if the different sequences exhibit similar physical properties. In the case of a nucleotide sequence, the different sequences will preferably have at least 50 percent, more preferably 70 percent, most preferably 90 percent or more sequence similarity between them. In the case of amino acid sequences, the different sequences have at least 70 percent, more preferably 80 percent, and most preferably 90 percent or more similarity between the polypeptides coded for by the amino acid sequences. Physical properties that may be similar include, for example, electrophoretic mobility, chromatography similarities, sediment gradient coefficients, spectrophotometric properties, etc.

Transcription Initiation Site: The nucleotide base in a DNA sequence of a gene at which mRNA transcription begins.

Unassociated Coding DNA Sequence: Part or all of a coding DNA sequence which is not naturally found with the regulatory region of the hybrid gene of which it is a part. As such, an unassociated coding DNA sequence would include a naturally occurring coding DNA sequence with a regulatory region different from the one with which it is naturally associated as well as a modified coding DNA sequence (altered from the natural DNA coding sequence by additions, deletions or substitutions of nucleotides in the sequence) with any regulatory region (including a regulatory region that may naturally be found with the coding DNA sequence in its unaltered or natural sequence). Preferably, an unassociated coding DNA sequence will have a regulatory region and a coding DNA sequence, each of which is naturally occurring individually but not naturally occurring together.

The present invention relates to certain hybrid DNA sequences having regulatory elements from a bacterial source and coding elements for pathogenesis related ("PR") proteins, cells containing such DNA sequences and methods for using such bacteria to produce an antimicrobial environment. For purposes of this invention, microbial is intended to include all microbial unicellular organisms, fungi, lichens, etc. In its broadest embodiment, the present invention relates to a DNA sequence comprising:

a) regulatory components from a bacterial source fused to

b) a DNA coding sequence from a plant, which DNA coding sequence results in expression of a plant pathogenesis related protein in a bacterium.

The regulatory elements from a bacterial source can be obtained from various sources including commercially available vectors, bacterial regulatory elements known in the art, and bacterial regulatory elements identified using a promoterless marker-containing transposon or promoter selection vectors such as pKK175-6 and pKK232-8 (Pharmacia, Piscataway, New Jersey). Commercially available bacterial regulatory elements are available from a number of sources such as the plasmid expression vectors pKK233-2, pDR540, pDR720, pYEJ001, pPL-Lambda (Pharmacia, Piscataway, New Jersey), pGEMEX expression vectors (Promega Biotec, Madison, Wisconsin).

Bacterial regulatory elements known in the art include any bacterial regulatory element that is known to function as a promoter, enhancer, ribosome binding site and/or any other regulatory control mechanism of the associated coding DNA sequence. An associated coding DNA sequence is a DNA sequence that is adjacent or adjoining 3'to the regulatory elements and which codes for a functional protein when transcribed and translated. Appropriate bacterial regulatory elements include those of Deretic et al. (1989); Deuschle et al. (1986); Hawley and McClure (1983); Rosenberg and Court (1979).

Bacterial regulatory elements identified using a promoterless marker-containing transposon can be obtained as follows:

An initial bacterial strain is first subjected to transposon mutagenesis. This bacterial strain is often a wild-type strain or it may be a strain having previous laboratory genetic alterations. Although it is not required for the practice of the invention, the bacterial strain is preferably in the form of a bacterial culture.

Preparation of the transposon that is used for mutagenesis may be by means well known in the art. Preferably, but not essentially, the transposon carries at least one marker gene to simplify subsequent bacterial identification and other manipulations. Useful markers include drug resistance, carbohydrate metabolism, light-generation, and ice nucleation genes. Examples of drug-resistance markers include resistance to kanamycin, neomycin, chloramphenicol, hygromycin, spectinomycin, tetracycline, and streptomycin, and examples of carbohydrate-metabolism marker genes include the genes for β-galactosidase and β-glucuronidase. One skilled in the art, however, can readily identify other examples of these types of markers as well as other types of markers useful in the practice of this invention. In a preferred embodiment of the method of this invention, the gene for β-galactosidase is used as the marker gene.

The marker gene is located near one end of the transposon and does not have a promoter. Expression of the marker gene depends on the presence of an active host bacterial gene promoter outside of the transposon and in the correct orientation with respect to the transposon. The transposon insertion mutants that show expression of the marker gene may be used to clone the bacterial promoters responsible for this expression using standard molecular biology techniques. To obtain the promoters, extraneous DNA is removed again using standard techniques, until the smallest region allowing expression of the marker gene is obtained. Thus, if the transposon is inserted in a bacterial gene that is normally expressed in response to a particular inducer, one will observe expression of the marker gene in the presence of the inducer, and no expression of the marker gene will be observed if the transposon is inserted in a gene having a promoter that is not turned on in the presence of that particular inducer.

After transposon mutagenesis, the mutagenized bacteria are subjected to induction screening to identify those mutants in which genes responsive to a plant host have been altered as a result of the mutagenesis. One skilled in the art can easily devise such a screen appropriate for the particular bacterium and marker. The transposon-insertion mutants that show a response to the inducer may be used to clone bacterial promoters of the corresponding genes using standard molecular biology techniques. To obtain the promoters, extraneous DNA is removed, again using standard techniques, until the smallest region that still responds to the inducer is obtained.

Any of the above can be synthesized using standard synthesis techniques (Applied Bio-systems). Bacterial regulatory elements include hybrid regulatory regions comprising mixtures of parts of the bacterial regulatory elements from different sources. For example, the tr/lac (trc) promoter of pKK232-2 (Pharmacia, New Jersey), which combines the -35 region of the *E. coli* tryptophan operon promoter with the -10 region of the *E. coli* lac operon promoter.

Sometimes such regulatory elements and coding DNA sequences are separated by a spacer or linker region such that the ribosome binding site of the regulatory DNA sequence and the ATG initiation codon of the DNA coding sequence are at an appropriate distance and orientation so as to provide efficient translation. Usually, the spacer or linker region is contained in the 3′end of the bacterial regulatory elements and is preferably from 3 to 15 nucleotide bases in length, most preferably about 9 nucleotide bases in length. Some of the spacer region may occur in the 5′ end of the DNA coding sequence so that after fusion the spacer region will occur intact directly 5′ to the ATG initiation codon.

Coding elements for pathogenesis related ("PR") proteins are for example described in EP 332, 104 or in the sequences SEQ ID NO:1 to SEQ ID NO:13.

An additional portion of the coding elements for PR proteins may include a signal sequence for a signal or leader peptide. This signal sequence, which is required for protein export, occurs 3′ to the ATG initiation codon and 5′ to the sequence that codes for the mature portion of the PR protein. Such signal sequences can be obtained from a variety of sources such as *Erwinia carotovora* (WO 89/06283; Better et al., 1988), *P. solanacearum* (Huang and Schell, 1990), *E. coli* (Sjöström et al., 1987), and others (von Heijne, 1985; von Heijne, 1984). A particularly useful signal peptide for use in *P.fluorescens* comprises the sequence from a *Pseudomonas* pectate lyase, SEQ ID NO:14.

In some (but not all) instances, significant bacterial expression and/or export of a plant PR protein may require replacement of the native plant signal sequence by an appropriate bacterial signal sequence or a mutant derivative of the plant signal sequence. In such instances, it is presumed that the native plant signal peptide interacts ineffectively with the bacterial export machinery, thereby interfering with normal cell export processes and placing the bacterium at a selective disadvantage. The substitution of a bacterial signal sequence such as the *P.fluorescens* signal sequence described above, or, for example, the mutant derivative of a tobacco basic β-1,3-glucanase signal sequence, SEQ ID NO:28, can enhance export and expression of active protein in such cases.

A first method of fusing of the relatory components from a bacterial source to a DNA coding sequence from a plant can occur using appropriate restriction sites. Preferably, the restriction site will overlap and contain the ATG initiation codon of the DNA coding sequence of the plant. More preferably, the restriction site will be a Nco site. Thus, one method for fusing plant gene cDNA sequences to the prokaryotic signals such that their native coding regions are translated requires creating an NcoI site by site-directed mutagenesis surrounding the cDNA start codon if an NcoI site does not already exist at this location. For example, in the expression of the cucumber chitinase, an NcoI site was created by site-directed mutagenesis of the CAATGG surrounding the gene's translation initiation codon to CCATGG.

A second more universally applicable method for fusing plant gene cDNA sequences to prokaryotic regulatory sequences takes advantage of the polymerase chain reaction overlap method of Horton et al. ( 1989). Briefly, a DNA segment containing the prokaryotic signals and a short overlap with the plant cDNA at the intended junction is amplified in one polymerase chain reaction. A DNA segment consisting of the plant cDNA and a short overlap with the prokaryotic transcription and translation signals at the intended junction is amplified in a second polymerase chain reaction. The overlap regions are allowed to hybridize and are amplified in a third polymerase chain reaction to yield the desired hybrid fragment. The polymerase chain reaction overlap extension protocol is known to those of skill in the art (Horton et al., 1989). The hybrid fragment is then ultimately cloned into a broad-host-range vector for introduction into bacteria, preferably Gramnegative bacteria, more preferably *Pseudomonas*. The best contemplated mode of making the invention involves a further step of introducing the hybrid fragment into the chromosome of the host bacterium for stable maintenance. Such introduction may be accomplished via homologous recombination between introduced sequences flanking the hybrid fragment and sites on the bacterial chromosome.

A third method of fusing plant gene cDNA sequences to prokaryotic regulatory sequences utilizes a combination of the PCR extension method and the restriction enzyme method. Briefly, a pair of PCR primers is used to generate a bacterial regulatory element wherein one of the primers contains both a segment complementary to a portion of the bacterial regulatory element and a portion of a plant DNA coding sequence having a suitable restriction site. Consequently, the PCR reaction will generate a bacterial regulatory element and a small portion of the 5′ end of the plant DNA coding sequence wherein the small portion of the 5′ end of the plant DNA coding sequence can be ligated to the remainder of the coding DNA using the restriction site contained within the small portion of the 5′ end of the DNA coding sequence.

The genetically engineered bacteria of the present invention can be used as an active ingredient in a soil compatible formulation or composition. Such bacteria applied in a soil compatible composition, particularly a seed coating, will colonize the plant's emerging roots, thereby affording protection against fungal pathogens. A preferred soil compatible composition is a seed coating. Examples of soil compatible formulations can be found in EP 320,483; US 4,875,921 and US 4,877,738.

The active ingredients of the present invention are normally applied in the form of compositions and can be applied to the crop area or plant to be treated, simultaneously or in succession, with further compounds. These compounds can be both fertilizers or micronutrient donors or other preparations that influence plant growth. They can also be selective herbicides, insecticides, fungicides, bactericides, nematicides, molluscicides or mixtures of several of these preparations, if desired, together with further carriers, surfactants or application-promoting adjuvants customarily employed in the art of formulation. Suitable carriers and adjuvants can be solid or liquid and correspond to the substances ordinarily employed in formulation technology, e.g. natural or regenerated mineral substances, solvents, dispersants, wetting agents, tackifiers, binders or fertilizers.

Preferably, the active ingredients may be applied to seeds (coating) by impregnating the seeds either with a liquid formulation containing active ingredients, or coating them with a solid formulation. The active ingredients can also reach the plant through the roots via the soil (systemic action) by impregnating the locus of the plant with a liquid composition, or by applying the compounds in solid form to the soil, e.g. in granular form (soil application). Another method of applying active ingredients of the present invention or an agrochemical composition which contains at least one of the active ingredients is leaf application. The number of applications and the rate of application depend on the intensity of infestation by the corresponding pathogen (type of fungus). In special cases, further types of application are also possible, for example, selective treatment of the plant stems or buds.

The active ingredients are used in unmodified form or, preferably, together with the adjuvants conventionally employed in the art of formulation, and are therefore formulated in known manner to emulsifiable concentrates, coatable pastes, directly sprayable or dilutable solutions, dilute emulsions, wettable powders, soluble powders, dusts, granulates, and also encapsulations, for example, in polymer substances. Like the nature of the compositions, the methods of application, such as spraying, atomizing, dusting, scattering or pouring, are chosen in accordance with the intended objectives and the prevailing circumstances. Advantageous rates of application are normally from 50 g to 5 kg of active ingredient (a.i.) per hectare (ha = 10 000 $m^2$), preferably from 100 g to 2 kg a.i./ha, most preferably from 200 g to 500 g a.i./ha.

The formulations, compositions or preparations containing the active ingredients and, where appropriate, a solid or liquid adjuvant, are prepared in known manner, for example by homogeneously mixing and/or grinding the active ingredients with extenders, for example solvents, solid carriers and, where appropriate, surface-active compounds (surfactants).

Suitable solvents include aqueous buffers, nutrient media, or water.

The solid carriers used e.g. for dusts and dispersible powders, are normally natural mineral fillers such as calcite, talcum, kaolin, montmorillonite or attapulgite, but can also include peat, methylcellulose, vermiculite or potting mixes. In order to improve the physical properties it is also possible to add highly dispersed silicic acid or highly dispersed absorbent polymers. Suitable granulated adsorptive carriers are porous types, for example pumice, broken brick, sepiolite or bentonite; and suitable nonsorbent carriers are materials such as calcite or sand. In addition, a great number of pregranulated materials of inorganic or organic nature can be used, e.g. especially dolomite or pulverized plant residues.

Depending on the nature of the active ingredient to be used in the formulation, suitable surface-active compounds are nonionic, cationic and/or anionic surfactants having good emulsifying, dispersing and wetting properties. The term "surfactants" will also be understood as comprising mixtures of surfactants.

Anionic surfactants that can be used include fatty sulfonates, fatty sulfates, sulfonated benzimidazole derivatives or alkylarylsulfonates.

The fatty sulfonates or sulfates are usually in the form of alkali metal salts, alkaline earth metal salts or unsubstituted or substituted ammoniums salts and have a 8 to 22 carbon alkyl radical which also includes the alkyl moiety of alkyl radicals, for example, the sodium or calcium salt of lignosulfonic acid, of dodecylsulfate or of a mixture of fatty alcohol sulfates obtained from natural fatty acids. These compounds also comprise the salts of sulfuric acid esters and sulfonic acids of fatty alcohol/ethylene oxide adducts. The sulfonated benzimidazole derivatives preferably contain 2 sulfonic acid groups and one fatty acid radical containing 8 to 22 carbon atoms. Examples of alkylarylsulfonates are the sodium, calcium or triethanolamine salts of dodecylbenzenesulfonic acid, dibutylnapthalenesulfonic acid, or of a naphthalenesulfonic acid/formaldehyde condensation product. Also suitable are corresponding phosphates, e.g. salts of the phosphoric acid ester of an adduct of p-nonylphenol with 4 to 14 moles of ethylene oxide.

Non-ionic surfactants are the water-soluble adducts of polyethylene oxide with polypropylene glycol, ethylenediamine propylene glycol and alkylpolypropylene glycol containing 1 to 10 carbon atoms in the alkyl chain, which adducts contain 20 to 250 ethylene glycol ether groups and 10 to 100 propylene glycol ether groups. These compounds usually contain 1 to 5 ethylene glycol units per propylene glycol unit.

Representative examples of non-ionic surfactants are nonylphenolpolyethoxyethanols, castor oil polyglycol ethers, polypropylene/polyethylene oxide adducts, tributylphenoxypolyethoxyethanol, polyethylene glycol and octylphenoxyethoxyethanol. Fatty acid esters of polyoxyethylene sorbitan and polyoxyethylene sorbitan trioleate are also suitable nonionic surfactants.

The surfactants customarily employed in the art of formulation are described, for example, in McCutcheon's Detergents and Emulsifiers Annual (1979) and Sisely and Wood (1980).

Sterilized seeds to be protected are coated with the candidate strains at a concentration of $10^6$ to $10^8$ bacteria/seed. This may be conveniently done by several ways. First, the bacteria are suspended in water containing 0.1 M $MgSO_4$ adjusting the population to $10^8$ to $10^9$ colony forming units (CFU), and soaking the seeds in the suspension for 30 min. Second, the bacteria are added to a suspension containing 0.5 to 2 % of a suspending agent such as methylcellulose in water (eight plates of bacteria per 50 ml methylcellulose solution per 100 g seed) and the seeds are dried ovemight.

The agrobacterial compositions usually contain from about 0.1 to about 99 %, preferably about 0.1 to about 95 %, and most preferably from about 3 to about 90 % of the active ingredient, from about 1 to about 99.9 %, preferably from about 1 to about 99 %, and most preferably from about 5 to about 95 % of a solid or liquid adjuvant, and froom about 0 to about 25 %, preferably about 0.1 to about 25 %, and most preferably from about 0.1 to about 20 % of a surfactant.

Whereas commercial products are preferably formulated as concentrates, the end user will normally employ dilute formulations.

## EXAMPLES

### Example 1

**Expression of cucumber chitinase gene in *P.fluorescens***

The procedure involves modification of cucumber chitinase gene cDNA by creation of a new restriction endonuclease site such that the cucumber chitinase translation start codon can be fused to a prokaryotic expression vector's transcription and translation regulatory sequences. Plasmids containing the appropriate fusion are introduced into *E. coli* by transformation and identified, following plasmid purification, on the basis of restriction endonuclease profile and DNA sequence of the fusion junction. The ability of the recombinant plasmid to confer chitinase activity upon *E. coli* is verified. This plasmid is further cloned into a broad host range plasmid for subsequent introduction by conjugation into *P.fluorescens*. A *P.fluorescens* transconjugant expressing chitinase activity is identified. The procedure for expressing cucumber chitinase in *P.fluorescens* has the following steps, described in detail below:

(a) Subcloning cucumber chitinase cDNA from pCUC6 into the bacteriophage M13 cloning vector mp 18.

A DNA fragment containing cucumber chitinase cDNA is released from pCUC6 (containing the cucumber chitinase coding sequence of ATCC 40528 in opposite orientation, which orientation can be inverted by digestion with *Eco*RI and subsequent ligation) by digestion with the restriction endonucleases *Sal*I and *Bam*HI. Ten units of each restriction enzyme are incubated in a 50 µl volume with approximately 3 µg of pCUC6 DNA in the presence of *Sal*I restriction buffer supplied by Promega Biotec. 1 µg of the cloning vector mp 18 is likewise digested with *Sal*I and *Bam*HI. The digested pCUC6 DNA is mixed with the digested mp 18 DNA in approximately a 5:1 ratio (ca. 0.5 µg pCUC6; ca. 0.1 µg mp 18) and ligated in a 20 µl volume in the presence of 50 mM Tris, pH 7.6; 10 mM $MgCl_2$; 10 mM dithiothreitol; 0.5 mM ATP; and 2 units T4 DNA ligase. Incubation is overnight at 15°C. Competent *E. coli* strain MV1190 are prepared according to the instructions of the supplier of a Muta-Gene M13 In Vitro Mutagenesis Kit (BioRad, Richmond, CA), and 300 µl of competent cells are mixed with approximately 10 ng of ligated DNA. Following incubation on ice for 45 min, the mixture is heat shocked at 42°C for 2 min and returned to ice. 100 µl of transgenic cells are mixed with 300 µl of an overnight culture of *E. coli* MV1190. To this mixture is added a mixture of 3.0 ml molten top agar (0.7 % w/v L agar), 50 µl 2.0 % X-gal dissolved in dimethyl formamide, and 20 µl 100 mM IPTG. The entire mixture is then vortexed and poured on an L agar plate. Stocks of potentially recombinant phage are recovered by removing clear plaques from the agar plate following overnight incubation and vortexing them in 1.0 ml 10 mM Tris, pH 8.0; 1 mM EDTA (TE buffer). Identification of recombinant phage is accomplished by subculturing a 3 ml overnight culture of *E. coli* MV1190 1:100 into tubes containing 3 ml of 2YT broth, incubating at 37°C for 2 hr, and adding 150 μl of each phage stock to different tubes. Growth is continued at 37°C for 5 hr, at which time the replicative form of each mp 18 derivative is purified by an alkaline lysis plasmid purification procedure (Maniatis et al., 1982). Approximately 0.1 μg of each plasmid is digested with *Bam*HI and *Sal*I as described above and clones containing the ca. 1.1 kb cucumber chitinase cDNA *Sal*I-*Bam*HI fragment are identified.

(b) Site directed mutagenesis of the subcloned cucumber chitinase cDNA with a mutagenic primer to create an *Nco*I restriction site at the chitinase translational start codon.

The DNA sequence immediately surrounding the translational start codon of the cucumber chitinase gene is 5′-CAATGG-3′(ATG start codon in bold type). Fusion of heterologous genes to the prokaryotic regulatory sequences of the expression vector pKK233-2 is simplified by the presence of an *Nco*I site (5′-CCATGG-3′) at the translational start codon to allow ligation with the appropriately positioned pKK233-2 *Nco*I site. An *Nco*I site is created at the start codon of the cucumber chitinase gene by using the single-stranded form of the recombinant mp 18 derivative of (a) as a template for a mutagenic primer of sequence SEQ ID NO:15, following procedures described in the BioRad Muta-Gene M13 in vitro mutagenesis kit:

A representative recombinant phage stock from step (a) above is titered on *E. coli* MV1190 by mixing serially diluted phage stock with 0.2 ml of overnight bacterial culture and 3 ml top agar and plating on L agar plates. After overnight incubation at 37°C, plaques are counted, and the phage stock is found to have a titer of 8.7 x $10^{13}$ plaque forming units (PFU) per ml. This phage is then grown on *E. coli* CJ236 (BioRad, Richmond, California), a strain which incorporates uracil into DNA, to obtain a uracil-containing template for the site-directed mutagenesis. This step provides a strong selection against the non-mutagenized strand of the phage. A subculture of *E. coli* CJ236 is allowed to grow to a Klett meter reading of approximately 20, corresponding to a viable cell count of 3.5 x $10^7$ CFU/ml. At this point, 4.5 x $10^7$ recombinant phage are added to 45 ml of *E. coli* CJ236, for a multiplicity of infection of 0.028. Bacteria are cultured at 37°C for 9 hr following the point of infection.

Phage particles are collected from 30 ml of culture supernatant following treatment with 150 μg RNAase by the addition of 1/4 volume 3.5 M ammonium acetate, 20 % PEG 8000; incubation on ice for 30 min; and centrifugation at 17,000 x g for 15 min at 4°C. The pellet is resuspended in 200 μl of high salt buffer (300 mM NaCl; 100 mM Tris, pH 8.0; 1 mM EDTA). This phage stock is titered on both *E. coli* CJ236 (uracil permissive) and *E. coli* MV1190 (BioRad, Richmond, California) (uracil non-permissive). The titer is 6.5 x $10^{12}$ PFU/ml on CJ236, and only 2.26 x $10^7$ PFU/ml on MV1190, indicating that uracil has been successfully incorporated into the phage.

Single-stranded DNA is recovered from phage particles by extracting the phage stock twice with an equal volume of neutralized phenol and once with phenol/chloroform ( 1:1). Then, chloroform extractions are continued until material is no longer visible at the interface between the aqueous and organic phases. One-tenth volume 7.8 M ammonium acetate and 2.5 volumes ethanol are mixed with the aqueous phase, incubated at -70°C for 30 min, and the sample is centrifuged at 4°C for 15 min. The pellet is washed in 90 % ethanol and dissolved in 20 μl TE buffer. A 2 μl aliquot is compared on an agarose gel with known quantities of single stranded phage phiX 174 DNA, and it is determined that the recombinant uracil-containing mp 18 derivative has a concentration of 0.4 μg/μl.

The mutagenic phage strand is synthesized by phosphorylating the mutagenic primer, annealing primer to the uracil-containing single-stranded template, and polymerizing the complementary DNA strand. For phosphorylation of the primer, 200 pmol of the mutagenic 25-mer are incubated with 4.5 units of T4 polynucleotide kinase in a 30 μl volume of 100 mM Tris, pH 8.0; 10 mM $MgCl_2$, 5 mM dithiothreitol, 0.4 mM ATP. Incubation is at 37°C for 45 min, and the reaction is stopped by heating at 65°C for 10 min. The annealing step is in a 10 μl volume of 20 mM Tris, pH 7.4; 2 mM $MgCl_2$, and 50 mM NaCl and included 0.1 pmol of uracil-containing template and 3 pmol of the mutagenic pier. The mixture is heated at 70°C and allowed to cool slowly to 30°C (by turning off the water bath) at which point it is placed on ice. The following ingrdients are then added to obtain the indicated final concentrations for synthesis of the complementary DNA strand: 0.4 mM of each of the four deoxyribonucleotide triphosphates; 0.75 mM ATP; 17.5 mM Tris, pH 7.4; 3.75 mM $MgCl_2$, 21.5 mM dithiothreitol, 5 units T4 DNA ligase, and 1 unit T4 DNA polymerase. The reaction is incubated on ice for 5 min, at 25°C for 5 min, and at 37°C for 90 min. At this point, 90 μl of stop buffer (10 mM Tris, pH 8; 10 mM EDTA) is added and the sample is frozen. Subsequently, competent *E. coli* MV1190 is transformed with the double stranded DNA. Resulting plaques are used as a source of phage for the generation of plasmid (replicative form) DNA preparations. The majority of such plasmids recovered contain the desired new *Nco*I site.

(c) Subcloning of the cucumber chitinase gene into the prokaryotic expression vector pKK233-2.

The cucumber chitinase gene is excised from the mutagenized mp 18 derivative by incubation with the restriction endonucleases *Nco*I and *Sma*I. Approximately 1 μg of the replicative form DNA obtained from (b) is digested at 30°C for three hr in a 30 μl volume containing *Sma*I buffer (Promega Biotec) and 10 units *Sma*I. The *Sma*I endonuclease is then removed by extraction with an equal volume of phenol:chloroform (1:1). The digested DNA is precipitated by the addition of 1/10 volume 3 M sodium acetate, pH 5.2, and two volumes ethanol, followed by incubation at -70°C for 30 min and centrifugation at 12,000 x g for 15 min. The DNA is then further digested with 10 units of *Nco*I for 3 hr at 37°C in a 30 μl volume of *Nco*I buffer (Promega).

*Nco*I is then removed by phenol:chloroform extraction and digested DNA is precipitated as above. 1 μg of the expression vector pKK233-2 (Pharmacia) is digested with 10 units of *Hind*III at 37°C in a 30 μl volume of *Hind*III buffer for 3 h. The 5′overhanging ends of the *Hind*III site are filled in to create blunt ends with the Klenow fragment of *E. coli* DNA polymerase by adding to the restriction digest 1 μl of a dNTP mixture (0.5 mM with respect to each of the four dNTPs) and 5 units of Klenow fragment. Incubation is at 30°C for 15 min. The reaction is stopped by heating at 75°C for 10 min followed by phenol:chloroform extraction and ethanol precipitation of the DNA. The vector is then digested with 10 units of *Nco*I at 37°C for 3 h in a 30 μl volume of *Nco*I buffer, followed by phenol:chloroform extraction and ethanol precipitation. Approximately 1 μg of both the digested pKK233-2 vector and the digested mp 18 derivative are electrophoresed through a 0.6 % low-melting-temperature agarose gel. Gel slices containing the vector figment and the *Nco*I-*Sma*I figment containing the cucumber chitinase gene are recovered and used in an in-gel ligation procedure (Crouse et al., 1983). Incubation is overnight at 15°C. 35 μl of diluted ligation mix (5 μl of melted DNA-containing agarose added to 30 μl 0.01 M Tris, pH 8.0; 0.01 M EDTA is then added to 200 μl of competent *E. coli* DH5 alpha Bethesda Research Laboratories, Gaithersburg, Maryland), held on ice for 45 min, heat shocked at 42°C for 90 sec, and incubated in 3 ml L broth at 37°C for 1 h. 200 μl aliquots of cells are plated to L agar supplemented with 100 μg/ml ampicillin.

(d) Plasmid purification, restriction endonuclease analysis, and DNA sequencing to verify the proper subcloning of the cucumber chitinase gene.

Ampicillin resistant colonies from (c) are screened for the presence of a plasmid larger than the original pKK233-2. Alkaline lysis plasmid preparations (Maniatis et al., 1982) are digested with *Nco*I and *Pst*I to verify the presence of the 1.1 kb cucumber chitinase gene-containing insert. Dideoxy DNA sequencing is performed on one such clone, designated pCIB3320. The DNA sequence indicates that no unwanted sequence changes occur in the vector's promoter or ribosome binding site. Likewise the fusion of vector sequences and cucumber chitinase DNA at the *Nco*I site is correct and no changes occur in the cucumber chitinase signal sequence region.

(e) Chitinase enzymatic assays.

*E. coli* DH5-alpha harboring pCIB3320 is grown in 300 ml L broth supplemented with 25 μg/ml ampicillin to an $OD_{600}$ of approximately 1.0 at which time 600 μl of 100 mM IPTG is added to ensure full induction of the *trc* promoter of the expression vector. Growth is continued for another 6 h, at which time cells are harvested by centrifugation at 5000 rpm. The cell pellet is washed once in 20 mM phosphate buffer (10 mM $Na_2HPO_4$/-10 mM $KH_2PO_4$), and, following centrifugation, the cell pellet is resuspended in 5 ml of the 20 mM phosphate buffer. Cells are lysed by sonication for 60 sec with the microtip of a Branson sonifier and cell debris is removed from the cell extracts by centrifugation.

Chitinase activity is assayed by incubation of 100 μl of cell extract with 100 μl of tritiated chitin (0.5 % w/v; approximately 0.1 mCi/ml) in a 250 μl total volume of 0.03 M sodium phosphate, pH 6.5, for 1 h at 37°C. The reaction is stopped by addition of an equal volume of 2 M TCA and centrifugation for 5 min in an Eppendorf centrifuge. 200 μl aliquots are counted in a liquid scintillation counter to determine soluble counts released from the insoluble chitin molecules as a result of chitinase activity. Table 1 contains results for *E. coli* cells containing or lacking the cucumber chitinase gene.

Table 1: Chitinase activity of *E. coli* DH5-alpha harboring pCIB3320

| | Counts per minute |
|---|---|
| *E. coli* DH5 alpha + pCIB3320 | 24,000 |
| *E. coli* DH5 alpha - pCIB3320 | 3,000 |

(f) Cloning of cucumber chitinase gene into the broad-host-range plasmid pLAFR3 for introduction into *P. fluorescens.*

pCIB3320 is digested with *Eco*RI by incubation of 1 µg of pCIB3320 for 3 h at 37°C with 10 units of EcoRI in a 50 µl volume of EcoRI buffer (Promega Biotec) to generate about a 1.4 kb EcoRI fragment containing the cucumber chitinase gene and about a 4.3 kb EcoRI fragment containing the remainder of the pKK233-2 expression vector. The broad-hostrange cloning vector pLAFR3 (Staskawicz et al., 1987) is likewise digested with *Eco*RI. Following phenol:chloroform extraction and ethanol precipitation of each DNA sample, a ligation reaction containing 0.5 µg of the digested pCIB3320 DNA, 0.1 µg of the digested pLAFR3 DNA, and 2 units of T4 DNA ligase in a 20 µl volume of 50 mM Tris, pH 7.6; 10 mM $MgCl_2$, 10 mM dithiothreitol, and 0.5 mM ATP is incubated overnight at 15°C. Competent *E. coli* DH5-alpha cells (200 µl) are added to the ligation reaction, incubated on ice for 45 min, heat shocked at 42°C for 2 min, and grown in 3 ml L broth at 37°C for 1 h. 200 µl aliquots are spread on L agar plates supplemented with 20 pg/ml tetracycline, 0.2 mM IPTG, and 0.004 % X-gal. After overnight incubation of the plates at 37°C, white colonies are isolated, and used as sources of bacteria for alkaline lysis plasmid preparations (Maniatis et al., 1982). Plasmid preparations are analyzed by digestion with *Eco*RI. The majority of the pLAFR3 recombinant derivatives consists of the about 4.3 kb *Eco*RI expression vector fragment cloned in pLAFR3. Only one recombinant contains the about 1.4 kb *Eco*RI fragment, and this fragment is accompanied in pLAFR3 by the 4.3 kb *Eco*RI fragment. This plasmid is designated pCIB3321. It is demonstrated that cloning of the 1.4 kb *Eco*RI fragment (which contains the strong *trc* promoter) likely requires the presence of the strong *rrn*B transcriptional terminators present in the 4.3 kb EcoRI fragment in the following experiment. The rrnB terminators are removed from pKK233-2 by digestion with X*mn*I and ligated with *Sma*I-digested pBluescript SK + (Stratagene). A pBluescript derivative containing the terminators is identified and digested with *Eco*RI and *Bam*HI to excise the terminators as an *Eco*RI-*Bam*HI fragment for ligation with EcoRI and *Bam*HI-digested pLAFR3. A pLAFR3 derivative containing the terminators is isolated, digested with *Eco*RI, and ligated with *Eco*RI-digested pCIB3320. Recombinants containing only the about 1.4 kb pCIB3320 *Eco*RI fragment bearing the cucumber chitinase gene can be recovered in this terminator-containing pLAFR3 derivative.

pCIB 3321 is further characterized by digestion with additional combinations of restriction endonucleases and found to include two copies of the 1.4 kb *Eco*RI fragment and two copies of the 4.3 kb *Eco*RI fragment in alternating order (i.e. 1.4, 4.3, 1.4, 4.3) cloned in the *Eco*RI site of pLAFR3. pCIB3321 is introduced into *E. coli* HB 101 by transformation, and conjugated into *P.fluorescens* strain 900 (NRL-B 15135, North Regional Laboratory, Peoria, Illinois) by the triparental mating procedure of Ditta et al. ( 1980). Briefly, overnight cultures of *E. coli* HB101 (pRK2013), *E. coli* HB 101 (pCIB3321), and *P.fluorescens* 900, (25 µl, 25 µl, and 200 µl, respectively) are mixed, spread on an L agar plate, and incubated at 28°C for 10 h. Loopfuls of bacteria are then streaked on *Pseudomonas* minimal media plates supplemented with 15 µg/ml tetracycline and incubated at 28°C overnight. Tetracycline resistant colonies are purified and screened for the presence of pCIB3321 by the alkaline lysis plasmid preparation procedure.

(g) Detection of cucumber chitinase activity in *P. fluorescens*

*P. fluorescens* strain 900 transconjugants harboring pCIB3321 are assayed for chitinase activity by the procedure described in section "e" above. Cells to be assayed are cultured in L broth supplemented with 15 µg/ml tetracycline. Table 2 presents results of chitinase assays on *P.fluorescens* 900 (pCIB3321).

Table 2: Chitinase activity of *P. fluorescens* harboring pCIB3321

| | Counts per minute |
|---|---|
| *P. fluorescens* 900 + pCIB3321 | 7,500 |
| *P. fluorescens* 900 - pCIB3321 | 3,500 |

(h) Immunoblotting to examine size of expressed cucumber chitinase in cell extracts and culture supernatants of *E. coli* and *P. fluorescens*.

Proteins recovered from cultures of *E. coli* (pCIB3320) and *P.fluorescens* (pCIB3321), as well as proteins recovered from supernatants of these cultures, are electrophoresed through 12 % SDS-polyacrylamide gels and electrophoretically transferred to nitrocellulose by the method of Burnette (1981). Approximately 100 µg of bacterial cell extract protein (prepared as described in section "e" above), or protein recovered from concentrated culture supernatants are loaded per lane of the SDS-polyacrylamide gels. Cucumber chitinase purified from plant tissue is also included on each gel as a standard. Nitrocellulose sheets containing the transferred proteins are placed in a blocking solution consisting of 1 % BSA, 0.02 % sodium azide, and 2 % lamb serum in phosphate buffer saline and incubated at room temperature for 30 min. The blots are then washed twice for 15 min each in wash buffer consisting of 10 mM Tris, pH 8.0; 0.05 % Tween 20; 0.02 % sodium azide; 1 % lamb serum; and 0.1 % BSA (fraction V). Antibody to cucumber chitinase (5 ml of either a 1:1000 or 1:3000 dilution of the polyclonal rabbit serum diluted in dot-blot diluent consisting of phosphate buffered saline containing 1 % BSA, 0.05 % Tween 20, 0.02 % sodium azide, and 10 % lamb serum) is added and incubation is at room temperature for 1 h. After two additional 15 min washes in room temperature wash buffer, 5 ml of 1 µg/ml goat anti-rabbit antibody in dot-blot diluent are added and incubation is for 1 h at room temperature. Blots are again washed twice for 15 min each in wash buffer, followed by addition of 5 ml of anti-goat antibody conjugated to alkaline phosphatase.

Incubation is for 1 h at room temperature. Two additional washes in wash buffer are followed by a 15 min room temperature wash in carbonate substrate buffer consisting of 0.84 % sodium bicarbonate, 0.53 % sodium carbonate, and 4 mM $MgCl_2$. 5 ml of a freshly-made substrate solution composed of 4.45 ml carbonate substrate buffer, 0.5 ml of a 1 mg/ml stock of p-nitroblue tetrazolium made up in carbonate substrate buffer, and 50 µl of a stock solution of 5 mg/ml bromo-chloro-indoyl phosphate made up in DMSO are added to each filter and incubated at room temperature for approximately 1 h to allow color development at the positions of immunoreacting protein bands. Color development is stopped with a water rinse. Two proteins reacting with cucumber chitinase antibodies are observed in cell extracts of *E. coli* (pCIB3320) and *P.fluorescens* (pCIB3321) - a processed form corresponding in size to the mature enzyme purified from cucumber and a larger, presumably unprocessed, form. The majority of the cucumber chitinase appearing in culture supernatants of either bacterium is in the processed form.

**Example 2**

**Expression of tobacco basic ββ-1,3-glucanase in *P. fluorescens***

Tobacco β-1,3-glucanase is expressed in *P.fluorescens* by taking the following steps, described in detail below.

(a) Polymerase chain reaction amplification of two DNA fragments, one containing pKK233-2 prokaryotic regulatory signals, the other containing the start codon of tobacco β-1,3-glucanase, and each containing an overlapping region at the desired fusion junction.

The polymerase chain reaction (PCR) overlap extension strategy of Horton et al. ( 1989) is used as a means of fusing the tobacco basic β-1,3-glucanase coding region to prokaryotic regulatory signals for bacterial gene expression. Two PCR primer oligonucleotides, SEQ ID NO: 16 and SEQ ID NO: 17, are used in the amplification of about 600 bp portion of the expression vector pKK233-2 which contains the bacterial *trc* promoter and a ribosome binding site. Two additional PCR primers, SEQ ID NO:18 and SEQ ID NO:19, are used in the amplification of an about 300 bp portion of the cDNA clone pCIB 1009 which contains the tobacco β-1,3-glucanase translation start codon and a 5′portion of the glucanase coding region.

pCIB 1009 is obtained from ATCC 40526 as follows: The plasmid pSGL2 is a subclone of the pGLN 17 cDNA (SEQ ID NO:20). This plasmid is digested with ClaI and EcoRI and the 1 kb fragment containing the glucanase cDNA is isolated from a LGT agarose gel. The pBluescript plasmid is digested with EcoRI, treated with CIAP and purified on a LGT agarose gel. The plasmid pBS-Gluc 39.1 contains a 4.4 kb insert which includes the glucanase coding sequence, about 1.5 kb of 5′ flanking sequence, a 600 bp intron, and about 1 kb of 3′ flanking sequence. This plasmid is used as a template in a PCR experiment containing two primers, SEQ ID NO:21 and SEQ ID NO:22. The result of this amplification is to produce a fragment to replace the truncated 5′ end of the glucanase cDNA. A single-base mutation creating an EcoRI site is introduced to facilitate cloning experiments. The PCR product is digested with EcoRI and ClaI and fragments are separated on a 2.0 % LGT agarose gel. A 120 bp band is excised, mixed with the 1 kb ClaI-EcoRI fragment from pSGL2 and the purified, EcoRI digested bluescript vector, ligated and transformed as described above. Transformants are screened for the presence of the insert and one plasmid with the proper structure is designated pCIB 1009.

Furthermore, the longest oligonucleotide of each pair includes a sequence which overlaps with the sequence of the longest oligonucleotide of the other pair, allowing the eventual fusion of the two fragments generated. Polymerase chain reactions are carried out in 100 μM volumes which are 200 μM with respect to each of the four deoxyribonucleotides, 1 μM with respect to each PCR primer, and containing, in reaction buffer supplied by the PCR kit manufacturer (Perkin Elmer Cetus), approximately 1 ng of plasmid DNA template and 2.5 units of Taq DNA polymerase. Cycle times and temperatures are, for the expression vector portion, 94°C for 45 sec for the denaturation step, 37°C for 60 sec for the annealing step, and 72°C for 90 sec for the extension step (with a 3 sec increase in this step per cycle). Times and temperatures for the glucanase cDNA clone portion are 94°C for 45 sec for the denaturation step, 45°C for 60 sec for the annealing step, and 72°C for 90 sec for the extension step (with a 3 sec/cycle extension step increase). Twenty-five cycles are routinely used. Amplified DNA fragments are recovered following chloroform extraction of the reaction by addition of 1/10 volume of 3 M sodium acetate pH 5.2, two volumes of ethanol, incubation at -70°C for 30 min, and centrifugation in an Eppendorf microcentrifuge at 12000 rpm.

(b) Fusion of the two amplified DNA fragments of (a) by the overlap extension protocol.

A 1: 10 dilution of each of the amplified DNA fragments recovered from (2a) is prepared and 1 μl of the fragment derived from the pKK233-2 template is combined with 3 μl of the fragment derived from pCIB 1009. In the presence of deoxyribonucletides (200 μM solution with respect to each), PCR reaction buffer, and Taq polymerase, these fragments are denatured at 94°C for 60 sec and allowed to anneal at 37°C for 2 min. Extension of one class of hybrid molecule (about 900 bases in length) anneals via the short overlapping region is accomplished at 72°C for 3 min to generate intact double-stranded fusion molecules. This series of incubations is repeated once. At this point, the two primers from (2a) which hybridize to the ends of the about 900 bp fusion molecule (SEQ ID NO:16 and SEQ ID NO:19) are added at a concentration of 1 μM. Thirty PCR cycles each consisting of a 50 sec, 94°C denaturation step, a 70 sec, 37°C annealing step, and a 120 sec (with 3 sec/cycle extension), 72°C extension step are carried out and the resulting amplified about 900 bp fusion fragment is visualized following electrophoresis through a 1 % agarose gel.

(c) Treatment of the amplified fusion fragment with T4 DNA polymerase to generate blunt ends.

The percentage of blunt ends in the population of amplified DNA fragments is increased by resuspending ethanol-precipitated fragment in T4 DNA polymerase buffer which is made 0.5 mM with respect to each deoxyribonucleotide and to which is added 10 units of T4 DNA polymerase. The 50 μl reaction is incubated at 11°C for 20 min. The reaction is stopped by phenol: chloroform extraction and the blunt-ended fragment is recovered by ethanol precipitation. Ligation reactions are performed with 1 μg of the fusion fragment and 0.1 μg of either pKK175-6 or pKK232-8 which has been digested with the restriction endonuclease *Sma*I. The former vector allows the recovery of promoter-containing fragments with selection for tetracycline resistance, while the latter vector allows the recovery of promoter-containing fragments with chloramphenicol resistance. Plasmid DNA is purified from *E. coli* containing pKK175-6 derivatives allowing growth on L agar supplemented with 0.2 mM IPTG and 5 μg/ml tetracycline, and from *E. coli* containing pKK232-8 derivatives allowing growth on L agar supplemented with 0.5 mM IPTG and 5μg/ml chloramphenicol.

(d) Restriction enzyme analysis of recombinant plasmids and DNA sequencing of fusion junction region.

Digestion of various pKK175-6 and pKK232-8 derivatives from (2c) with the restriction endonucleases *Sal*I and *Cla*I, which are predicted to cut within the desired insert, is performed. The DNA sequence of the fusion fragment jonction region of several recombinant plasmids is determined by Sanger dideoxy sequencing of double-stranded templates to determine whether the overlap extension protocol has resulted in the desired fusion junction. One pKK175-6 derivative with the desired fusion of the tobacco β-1,3-glucanase translation start codon to the ribosome binding site region of the pKK233-2*trc* regulatory sequences is selected for further manipulation. It should be noted that the PCR amplification process results in a single base change in the 28th amino acid of the β-1,3-glucanase pre-protein, but that this change does not affect the amino acid sequence of the mature β-1,3-glucanase molecule.

(e) Subcloning the remainder of the tobacco basic β-1,3-glucanase gene

The plasmid pGLN17 is a hybrid cDNA encoding the basic β-1,3-glucanase from *N. tabacum* (Shinshi et al., 1988) constructed by fusing the 5′end of the pGL31 clone and the 3′ end of the pGL36 clone. The sequence encoded in this hybrid cDNA is SEQ ID NO:20.

pGLN17 is digested with the restriction endonucleases *Cla*I and *Hind*III to release a about 1.2 kb DNA fragment containing the remainder of the glucanase coding region 3′ of the *Cla*I site. The pKK175-6 derivative of (2d) with the fusion fragment insert likewise is digested with *Cla*I and *Hind*III. The about 1.2 kb pGLN17 *Cla*I-*Hind*III fragment is ligated with the digested pKK175-6 derivative, a step which serves to join the remainder of the β-1,3-glucanase coding region to the fusion fragment at the *Cla*I site within the β-1,3-glucanase gene. An *E. coli* transformant containing the intact β-1,3-glucanase gene fused to prokaryotic regulatory signals is identified following alkaline lysis plasmid purification, digestion of plasmid DNA with the restriction endonucleases *Cla*I and *Hind*III, and electrophoresis of digested DNA through a 0.7 % agarose gel. The desired plasmid is preliminarily designated pTC6::GLN17-3.

(f) Assay for β-1,3-glucanase activity in transformants containing the intact gene.

*E. coli* DH5 alpha harboring pTC6::GLN17-3 is assayed for the production of β-1,3-glucanase activity by growing a 300 ml culture at 30°C in L-broth supplemented with 25 μg/ml ampicillin to an optical density at 600 nm of 1.2, adding 600 μl of 100 mM IPTG to the culture at that point and continuing incubation for an additional 12 h. Cells are harvested by centrifugation for 5 min at 500 rpm, washed once in 50 ml 20 mM phosphate buffer, and resuspended following cetrifugation in 5 ml of 20 mM phosphate buffer. Cells are disrupted by sonication for 75 sec with the microtip of a Branson sonicator, and cell debris is removed by centrifugation for 15 min at 10,000 rpm. The bacterial extracts are incubated at room temperature for 24 hours to inactivate any *E. coli* enzymes which might act upon products generated by β-1,3-glucanase activity. Extracts are assayed for β-1,3-glucanase activity essentially by the procedure of Kombrink et al. (1985). Enzyme assay reaction mixtures contain, in 1.0 ml total volume, 100 μl bacterial extract, 2.0 mg of the β-1,3-glucan laminarin, and 100 mM potassium acetate, pH 5.3. At timed intervals, 200 μl of each reaction mixture is added to 3.0 ml of a 0.5 % solution of the colorimetric reagent p-hydroxybenzoic acid hydrazide and heated in boiling water for 6 min. After cooling on ice, the absorbance of each sample is read at 410 nm and compared with the absorbance of a control sample lacking enzyme. Representative β-1,3-glucanase activities are presented in Table 3.

Table 3: β-glucanase activities

| | β-1,3-glucanase activity [U]* |
|---|---|
| *E. coli* DH5 alpha + pTC6::GLN17-3 | 23.2 |
| *E. coli* DH5 alpha - pTC6::GLN17-3 | 0.29 |
| *P. fluorescens* 900 + pCIB3306 | 4.7 |
| *P. fluorescens* 900 - pCIB3306 | 0.25 |

* U = nmol glucose equivalents released from the laminarin (β-1,3 glucan) substrate per minute per milligram protein in the bacterial cell extract

(g) Subcloning of the β-1,3-glucanase gene into the broad-host-range plasmid pLAFR3 for introduction by conjugation into *P.fluorescens*.

The β-1,3-glucanase expression construction is subcloned from pTC6::GLN17-3 into pKK232-8 by ligation of a about 1.5 kb *Bam*HI-*Hind*III fragment from pTC6::GLN17-3 (containing the trc regulatory sequences and the β-1,3-glucanase coding region) with *Bam*HI and *Hind*III-digested pKK232-8. This pKK232-8 derivative is then digested with *Bam*HI, ligated with *Bam*HI-digested pLAFR3, and transformed into *E. coli* DH5-alpha. Potential transformants are picked as white colonies on L agar plates supplemented with 15 μg/ml tetracycline, 0.2 mM IPTG, and 40 μg/ml Xgal. A pLAFR3 derivative containing the β-1,3-glucanase construction is first introduced into *E. coli* HB 101 by transformation and then into *P.fluorescens* 900 by conjugation from its *E. coli* HB 101 host using the triparental mating procedure.

(h) Assay for β-1,3-glucanase activity in *P.fluorescens* transconjugants.

*P.fluorescens* 900 harboring the pLAFR3 derivative containing the tobacco basic β-1,3-glucanase expression construct (pCIB3306) is assayed for β-1,3-glucanase activity by the procedure of (2f). Results are presented in Table 3.

**Example 3**

**Expression of tobacco basic chitinase in *P.fluorescens***

Tobacco basic chitinase has thus far been expressed in *P.fluorescens* as a fusion protein consisting of amino terminal amino acids encoded by the *lac*Z alpha peptide portion of the multicloning site of pLAFR3 (up to the *Bam*HI site), codons specified by a normally untranslated portion of a tobacco chitinase cDNA clone [pCHN89, which is essentially identical to pSCH10 containing a cDNA insert of the tobacco basic chitinase which is similar to the insert in pCHN50 (Shinshi et al., 1987) but with an extension of 81 bp on the 5′end. The 80 extra bp are SEQ ID NO:23 between a *Bam*HI site and the usual translational start codon, and finally the entire basic chitinase coding region. Thus the fusion gene contains an additional 21 codons of non-chitinase gene DNA at its 5′end. Steps in expressing tobacco basic chitinase in *P.fluorescens*, described in detail below, are as follows.

(a) Subcloning the tobacco chitinase gene from pCHN89 as a *Bam*HI-*Hind*III restriction fragment into the broad-host-range plasmid pLAFR3 and introduction by transformation into *E. coli*. Restriction enzyme analysis of plasmid DNA purified from transformants.

The broad-host-range plasmid pLAFR3 is digested with the restriction endonucleases *Bam*HI and *Hind*III in a 200 μl volume containing: 1 μg pLAFR3, 20 μl 10 x *Bam*HI restriction buffer (Promega Biotec), 20 units of *Bam*HI and 20 units of *Hind*III. Approximately 10 μg of the tobacco basic chitinase cDNA clone pCHN89 is digested with the same enzymes in a 100 μl volume. Incubation of each restriction digest is at 37°C overnight. Each sample is phenol:chloroform extracted once and then precipitated by the addition of 1:10 volume of 3 M sodium acetate, pH 5.2 and two volumes of ethanol, followed by incubation at -80°C for 20 min and centrifugation at 4°C for 15 min. DNA pellets are washed once with 70 % ethanol and resuspended in 20 μl distilled water. The two samples are ligated in a 30 μl volume containing 50 mM Tris, pH 7.6; 10 mM $MgCl_2$, 10 mM dithiothreitol, 0.5 mM ATP, 2 units T4 DNA ligase, and 3 μl of each of the DNA samples. Incubation is at 15°C overnight.

The entire ligation reaction is added to 200 μl of competent *E. coli* DH5-alpha, held on ice 40 min, and heat shocked at 42°C for 90 sec. The cells are then added to 3 ml L broth and incubated at 37°C for 2 h. 200 μl aliquots are then spread on L agar plates containing 15 μg/ml tetracycline, 0.2 mM IPTG, and 0.004 % X-gal. Plates are incubated at 37°C overnight, and white colonies are saved on L agar plates containing 15 μg/ml tetracycline.

Plasmid DNA is purified from these white colonies by inoculating a loopful of each into 3 ml L broth supplemented with 10 μg/ml tetracycline, incubating at 37°C overnight, and performing the alkaline lysis procedure (Maniatis et al., 1982). Approximately 0.1 μg of plasmid DNA from four samples is digested in a 50 μl reaction with *Bam*HI and *Hind*III . Agarose gel electrophoresis reveals that each of the plasmids has acquired the about 1.2 kb *Bam*HI-*Hind*III fragment containing the tobacco basic chitinase gene.

(b) Assay for chitinase activity in *E. coli* harboring the recombinant plasmid of Example 3(a).

*E. coli* cells harboring pCIB3310 are assayed for chitinase activity by the procedure described in Example 1(e). Results are presented in Table 4.

(c) Conjugation of the recombinant plasmid into *P.fluorescens.*

pCIB 3310 purified from *E. coli* DH5-alpha in Example 3(a) is used to transform competent *E. coli* HB101. pCIB3310 is mobilized from *E. coli* HB 101 to *P. fluorescens* 900 by conjugation the triparental mating procedure.

(d) Assay for chitinase activity in a *P.fluorescens* transconjugant.

*P.fluorescens* 900 cells harboring pCIB3310 are assayed for chitinase activity by the procedure described in Example 1 (e). Results are presented in Table 4.

Table 4: Chitinase activity

| | Counts per minute |
|---|---|
| *E. coli* DH5 alpha + pCIB3310 | 7,500 |
| *E. coli* DH5 alpha - pCIB3310 | 2,500 |
| *P.fluorescens* 900 + pCIB3310 | 7,500 |
| *P.fluorescens* 900 - pCIB3310 | 3,750 |

**Example 4**

**Additional PR protein genes expression in root-colonizing bacteria**

This invention can be extended to the bacterial expression of complementary DNA (cDNA) clones obtained from any plant source in which such PR proteins are synthesized in response to pathogen attack or chemical treatment. In addition to the cucumber chitinase/lysozyme, tobacco basic glucanase, and tobacco chitinase described in examples 1 to 3, a variety of other PR proteins from both dicotyledonous and monocotyledonous plants have been described. In tobacco, these include PR-1A, PR-1B, PR-1C, PR-R major, PR-R minor, PR-P, PR-Q, PR-Q′, PR-2, PR-N, PR-O, and PR-O′. In tomato, these include p14, W, X, and Y (Carr and Klessig, 1989). In potato, a cDNA encoding a basic chitinase has been identified (Gaynor, 1988), and several potato PR proteins have been identified as chitinases and β-1,3-glucanases (Kombrink et al., 1985). In bean, a basic chitinase and a basic β-1,3-glucanase have been identified (Vögeli et al., 1988). In pea, two basic chitinases, Ch1 and Ch2, and two basic β-1,3-glucanases, G1 and G2, have been isolated (Mauch et al., 1988b). In maize, eight PR proteins, PRm 1 to PRm 8, have been identified (Nasser et al., 1988). A β-1,3-glucanase has been identified in *Arabidopsis thaliana* (Davis and Ausubel, 1989).

The following steps are those which would be required for the expression of additional PR proteins in *E. coli* and *P.fluorescens*:

a) Treatment of plant or plant tissue with pathogen or chemical inducer (ex. salicylic acid) followed by isolation of cDNA clones encoding PR proteins.

The treatment of a plant or plant tissue with a chemical inducer (ex. salicylic acid) followed by isolation of cDNA clones is described in EP 332, 104.

Treatment of a plant or plant tissue with a pathogen followed by isolation of cDNA clones encoding PR proteins is described below:

A plant or plant tissue is infected with a pathogen and RNA is isolated 5 days after infection as described above. Poly A+ RNA is isolated by standard techniques and a cDNA library is constructed in the lambda Zap cloning vector (Stratagene), essentially as described (Gubler and Hoffman, 1983).

Plaques from the cDNA library are screened with an oligonucleotide of the sequence SEQ ID NO:24. Positive plaques are purified by standard techniques and DNA is prepared from the phage. A fragment of the recombinant phage which contains the cDNA is subcloned into the bluescript plasmid. A partial cDNA sequence of each clone is determined by DNA sequencing.

b) Subcloning of coding region from cDNA of (a) behind a bacterial promoter and ribosome binding site such that the translation initiation codon of the PR protein gene is situated at or near an optimal distance (between 3 and 15 bp and preferably between 8 and 12 bp) from the bacterial ribosome binding site.

One possible method for accomplishing this fusion between bacterial and plant DNA sequences involves generating a common restriction endonuclease site by site-directed mutagenesis both at the appropriate position downstream (3′) of the bacterial ribosome binding site and at the plant cDNA translation initiation codon (such that the initiation codon is included within the restriction site). As an example, the translation initiation codon sequence ATG is included within the restriction endonuclease site *Nco*I (CCATGG) and within the restriction endonuclease site *Nde*I (CATATG). Thus, a bacterial regulatory region containing an *Nde*I site, for example, 8 bases downstream (3′) of its ribosome binding site and a different restriction site X further downstream could be fused to a plant PR protein cDNA sequence containing an *Nde*I site at its translation initiation codon and restriction site X further downstream by digesting a bacterial plasmid vector containing the bacterial regulatory region both with *Nde*I and restriction enzyme X and ligating with the PR gene cDNA fragment obtained following digestion with NdeI and restriction enzyme X. In this example, if the entire PR gene coding region should not be contained within the *Nde*I-X fragment, it could be added in additional subcloning steps (see also example 1 of this application, in which a cucumber chitinase cDNA coding region is fused to bacterial regulatory sequences following generation of an NcoI site overlapping the translation initiation codon of the cucumber chitinase gene. In this example, an *Nco*I site is already present at an appropriate distance downstream of the bacterial ribosome binding site.)

Another possible method for fusing bacterial regulatory sequences to plant PR gene coding regions employs the polymerase chain reaction strategy (Horton et al., 1989). (See example 2 of this application for details of how this procedure is used to obtain bacterial expression of a tobacco basic β-1,3-glucanase). It should be noted that either of these methods can be used to fuse truncated or extended versions of plant PR coding regions to bacterial regulatory regions simply by positioning with these methods a translation initiation codon at a position other than its original one. Likewise, as discussed in example 5 below, it is also possible to fuse the sequences which encode the mature portion of a given plant PR protein (i.e lacking the sequences which encode the leader peptide portion of the PR protein) to bacterial sequences which include a promoter, ribosome binding site and which encode a bacterial leader peptide such that the coding region for the mature PR protein follows in proper frame the bacterial leader peptide coding region.

c) Introduction of the engineered PR gene from (b) into *P.fluorescens*.

A DNA restriction fragment or fragments containing bacterial regulatory (and possibly leader) sequences fused to a particular plant PR protein coding region can be subcloned into the multicloning site of the broad-host-range plasmid pLAFR3 and introduced by conjugation into *P.fluorescens* (see examples 1 to 3 of this application) or alternatively by transformation using an electroporation apparatus. Another approach would involve subcloning the fusion construction into a transposable element containing a second marker gene and introducing a plasmid vector incapable of replicating in *Pseudomonas* and containing this transposable element derivative either by conjugation, transformation, or transduction into *P.fluorescens.P.fluorescens* recipients containing the transposable element can be screened on the basis of having acquired the second marker gene. Another approach would involve subcloning the fusion construction and a second marker gene between originally contiguous portions of the *P.fluorescens* chromosome which had previously been cloned into an *E. coli* plasmid incapable of replicating in *Pseudomonas*. Such a plasmid could be introduced into *P.fluorescens* by conjugation, transformation, or transduction, and *P.fluorescens* recipients acquiring the fusion construction by homologous recombination involving the external *P.fluorescens* chromosomal sequences could be identified on the basis of their acquisition of the marker gene.

d) Detection of expression of PR proteins in bacteria.

When the particular PR protein selected for expression has a known enzymatic activity such as chitinase or β-1,3-glucanase, such activities can be assayed in bacterial cell extracts (for details, see examples 1 and 2 of this application). When the function of a given PR protein is unknown, detection of bacterial expression would require generation of antibody to the PR protein purified from its plant source or from transgenic plants expressing such protein, and use of an immunoblotting protocol (such as the protocol described in example 1, part h).

## Example 5

**Fusion of DNA sequences encoding mature portion of a tobacco basic β-1,3-glucanase to bacterial (*P. fluorescens*) DNA sequences encoding ribosome binding site and pectate lyase leader peptide**

A hybrid gene consisting of a bacterial ribosome binding site and bacterial leader peptide sequence (a *P.fluorescens* pectate lyase leader peptide sequence) fused to sequences encoding the mature portion of a tobacco basic β-1,3-glucanase is constructed as described below.

(a) Polymerase chain reaction amplification of a DNA fragment containing the ribosome binding site of a *P.fluorescens* pectate lyase gene, the leader peptide sequence of a *P.fluorescens* pectate lyase gene, and the 5′ portion of the coding region for the mature portion of a tobacco basic β-1,3-glucanase gene.

The polymerase chain reaction (Saiki et al., 1988) is used to amplify a novel hybrid DNA fragment which contains sequences from the 5′ portion of the region encoding the mature tobacco basic β-1,3-glucanase (i.e. the sequences immediately 3′ of the tobacco basic β-1,3-glucanase signal sequence) fused in the correct reading frame to sequences encoding a *P.fluorescens* pectate lyase gene ribosome binding site and leader peptide. Two PCR primer oligonucleotides, the 16-mer SEQ ID NO:25 and the 30-mer SEQ ID NO:26 are used in the amplification of this ca. 500 bp hybrid fragment from a template consisting of a *P.fluorescens* pectate lyase gene cloned on a ca. 1.7 kb fragment in pUC18. The 16-mer anneals to its complementary sequence in pUC18 and directs the polymerization of DNA molecules toward the *Eco*RI site of the pUC18 multicloning site. The 30-mer contains 16 bases at its 3′ end (AGCATGCGCAGGAACG) which anneal with the final 16 bases of the sense strand of the *P.fluorescens* pectate lyase leader peptide sequence and direct the polymerization of DNA molecules toward the translation start codon of the pectate lyase gene and ultimately toward the site at which the first primer has annealed. The additional 14 bases (ACACCTATCGATTG) at the 5′ end of this 30-mer are complementary to the first 14 bases of the sense strand of DNA encoding the mature portion of a tobacco basic β-1,3-glucanase and include a naturally occurring *Cla*I site (ATCGAT) present at this position in the glucanase gene. Thus, the amplified DNA figment generated from the polymerase chain reaction using the primers and template described above contains the first 14 bp of the mature glucanase coding region (including the naturally occurring *Cla*I site) fused to a *P.fluorescens* pectate lyase gene ribosome binding site and leader peptide sequence.

Polymerase chain reactions using the primer oligonucleotides described above are carried out in 100 μl volumes which are 200 μM with respect to each of the four deoxyribonucleotides, 1 μM with respect to each PCR primer, and containing, in reaction buffer supplied by the PCR kit manufacturer (Perkin Elmer Cetus), approximately 1 ng of plasmid DNA template and 2.5 units Taq DNA polymerase. Cycle times and temperatures are 94°C for 45 sec for the denaturation step, 43°C for 60 sec for the annealing step, and 72°C for 90 sec for the extension step (with a 3 sec/cycle extension step increase). Twenty-five cycles are used. Amplified DNA fragments from eight such polymerase chain reactions are pooled and recovered, following chloroform extraction of the reaction, by addition of 1/10 volume of 3 M sodium acetate, pH 5.2, two volumes of ethanol, incubation at -70°C for 30 min, and centrifugation in an Eppendorf microcentrifuge at 12000 rpm.

(b) Subcloning of the amplified hybrid fragment into the *E. coli* vector pBluescript SK II (+).

Approximately 2 μg of the amplified DNA fragment of (a) is digested with 10 units each of the restriction endonucleases *Asp*718 and *Cla*I. *Asp*718 cut once within the amplified fragment at a position upstream (5′) of the pectate lyase gene ribosome binding site, while *Cla*I cut once near one end of the amplified fragment at a position within the coding region for the mature tobacco basic β-1,3-glucanase. (Note: This *Cla*I site is contained within the 30-mer PCR primer as described in (a) above). Approximately 1 μg of this ca. 180 bp *Asp*718-*Cla*I fragment, SEQ ID NO:27, is ligated with approximately 0.1 μg of pBluescript SK II(+) which had been similarly digested with *Asp*718 and *Cla*I. The ligation reaction (30 μl) is added to 200 μl of competent *E. coli* DH5 alpha, held on ice for 45 min, heat shocked at 42°C for 90 sec, and incubated in 3 ml L broth at 37°C for 1 h. 200 μl aliquots of cells are plated to L agar supplemented with 100 μg/ml ampicillin, 0.2 mM IPTG, and 0.004 % X-gal. After overnight incubation at 37°C, white colonies are isolated and used as sources of bacteria for alkaline lysis plasmid preparations. Recovered plasmid samples are analyzed by digestion with *Asp*718 and *Cla*I. A representative plasmid preparation with the desired restriction endonuclease profile is purified by isopycnic cesium chloride/ethidium bromide centrifugation for DNA sequence analysis. DNA sequence is determined for each strand of the insert by the dideoxy method using both the m13/pUC -20 and reverse sequencing primers and double-stranded plasmid template. A plasmid whose pectate lyase leader/tobacco basic β-1,3-glucanase hybrid fragment has the correct sequence is used in (c) below as the starting material for the addition of the remainder of the tobacco basic β-1,3-glucanase coding region.

(c) Subcloning of the remainder of the tobacco basic β-1,3-glucanase coding region into the recombinant pBluescript SK II(+) derivative of (b) above.

pBluescript SK II(+) containing the ca. 180 bp *Asp*718-ClaI insert of (b) is digested with the restriction endonucleases *Cla*I and *Pst*I. *Cla*I cut this plasmid once at the end of the insert within the tobacco basic β-1,3-glucanase sequence (i.e. downstream of the fusion between pectate lyase leader sequences and glucanase sequences). *Pst*I cut this plasmid once within the pBluescript multicloning site at a position further downstream of the *Cla*I site. The tobacco basic β-1,3-glucanase cDNA clone pCIB1009 is digested with *Cla*I and *Pst*I to release a ca. 1.2 kb *Cla*I-*Pst*I fragment consisting of the remainder of the coding region for the mature β-1,3-glucanase not already present in the aforementioned ca. 180 bp *Asp*718-*Cla*I fragment. This 1.2 kb *Cla*I-*Pst*I fragment is ligated with the *Cla*I and *Pst*I-digested pBluescript SK II(+) derivative of (b), and the ligation reaction is used to transform competent *E. coli* DH5 alpha. pBluescript SK II(+) derivatives with the complete coding region for the mature portion of tobacco basic β-1,3-glucanase fused to the pectate lyase leader peptide (and positioned behind the pectate lyase gene ribosome binding site) are recovered by the standard procedures described in (b) above. One such plasmid is designated pCIB3305. pCIB3305 contains the hybrid gene oriented opposite the lac promoter of the pBluescript SK II(+) vector. It should be noted that the hybrid gene may be excised from this vector as, for example, an *Asp*718-*Pst*1 fragment for subcloning behind an appropriate bacterial promoter (for example, the *Asp*718-*Pst*1 fragment could be ligated with *Asp*718 and *Pst*I-digested pUC 18 to position the hybrid construct behind the pUC18 *lac* promoter).

**Example 6:**

**Fusion of DNA sequences encoding mature portions of cucumber chitinase to bacterial (*P.fluorescens*) DNA sequences encoding the ribosome binding site and the pectate lyase leader peptide**

A hybrid gene consisting of a bacterial ribosome binding site and bacterial leader peptide sequence fused to a sequence encoding the mature portion of a cucumber chitinase is constructed by a strategy essentially the same as that used in Example 5. The differences are described below:

(a) The two PCR primer oligonucleotides used to amplify a novel hybrid DNA fragment, containing sequences from the 5′ portion of the region encoding the mature cucumber chitinase (i.e. the sequences immediately 3′ of the cucumber chitinase signal sequence) fused in the correct reading frame to sequences encoding a *P.fluorescens* pectate lyase gene ribosome binding site and leader peptide, are SEQ ID NO:29 and SEQ ID NO:30. As in Example 5, SEQ ID NO:29 anneals to its complementary sequence in pUC18 and directs the polymerization of DNA molecules toward the *Eco*RI site of the pUC18 multicloning site. SEQ ID NO:30 contains 16 bases at its 3′ end (AGCATGCGCAGGAACG) which anneal with the final 16 bases of the sense strand of the *P.fluorescens* pectate lyase leader peptide sequence and directs the polymerization of DNA molecules toward the translation start codon of the pectate lyase gene and ultimately toward the site at which the first primer has annealed. The additional 25 bases of SEQ ID NO:30 at the 5′ end of this 41-mer are complementary to the first 25 bases of the sense strand of DNA encoding the mature portion of cucumber chitinase and include a naturally occurring *Pfl*MI site (CCAATAGATGG) present at this position in the cucumber chitinase gene. Thus, the amplified DNA fragment generated from the polymerase chain reaction using these two primers contains the first 25 base pairs of the mature cucumber chitinase coding region (including the naturally occurring *Pfl*MI site) fused to a P.*fluorescens* pectate lyase gene ribosome binding site and leader peptide sequence.

(b) The amplified fragment of (a) is cloned into the plasmid vector pKK232-8 (Pharmacia) by treating the fragment with T4 DNA polymerase in the presence of deoxyribonucleotides to generate flush ends and ligating with *Sma*I-digested pKK232-8. Transformants containing the amplified fragment in the desired orientation are identified on the basis of expression of the pKK232-8 chloramphenicol resistance gene which is dependent upon the *lac* promoter of the amplified fragment. The amplified fragment is further subcloned into the plasmid vector pUC 19 by releasing it from pKK232-8 by digestion with *Eco*RI and PstI and ligating it with similarly digested pUC19.

(c) The remainder of the cucumber chitinase coding region is subcloned into the recombinant pUC19 derivative of (b) above by ligating the gel-purified ca. 1 kb *Pfl*MI-*Pst*I fragment of the cucumber chitinase cDNA clone pCUC6 with the *Pfl*MI- and *Pst*I-digested pUC19 derivative. One plasmid recovered from such a ligation is designated pCIB3313. pCIB3313 contains the hybrid gene oriented opposite the *lac* promoter of the pUC 19 vector. It should be noted that the hybrid gene may be excised from pCIB3313 as, for example, an *Eco*RI fragment for subcloning behind an appropriate bacterial promoter (as for example, pCIB3313 is itself digested with *Eco*RI and religated to obtain a derivative in which the fusion gene is oriented behind the pUC 19 *lac* promoter. This construct is designated pCIB3314).

**Example 7:**

**Control of *Rhizoctonia solani* post-emergence damping-off of cotton with transgenic *P.fluorescens* expressing plant pathogenesis-related proteins**

Transgenic *P.fluorescens* strain 900 derivatives expressing either cucumber chitinase (Example 1) or tobacco basic β-1,3-glucanase (Example 2), or a mixture of each such transgenic strain, are applied to cotton seeds to provide protection against post-emergence cotton damping-off caused by the fungal pathogen *Rhizoctonia solani*.

(a1 ) Addition of *R. solani* inoculum to potting mixture: A 1:1 mix of sand and vermiculite is autoclaved on each of two successive days for 70 min at 250°C. *R.solani*-infested millet seed is added in a large drum mixer at a rate of 5 mg/180 ml potting mixture. 50 ml of sterile distilled water is present per 180 ml of potting mixture.

(a2) Application of transgenic *P.fluorescens* strains to cotton seeds:*P.fluorescens* strains are inoculated into 50 ml of L broth supplemented with 12 μg/ml tetracycline and grown overnight with shaking at 28°C. Strains used in this experiment are *P.fluorescens* 900 (pCIB3321) (from Example 1), *P.fluorescens* 900 (pCIB3306) (from Example 2), and *P.fluorescens* 900 (pLAFR3) (a control strain containing the unmodified broad-host-range vector used in the construction of the recombinant plasmids pCIB3321 and pCIB3306). The optical density at 600 nm of a portion of each culture is adjusted by dilution to 0.2. In addition, one diluted culture is prepared such that it contains a 1:1 ration of *P.fluorescens* 900 (pCIB3321) and *P.fluorescens* 900 (pCIB3306). Approximately 40 cotton seeds (variety Coker 310, mechanically delinted) are added to each diluted culture and allowed to soak for a minimum of 10 min. Thirty seeds from each treatment are planted one seed per pot at a depth of approximately 2.5 cm in pots containing approximately 180 ml each of *R. solani*-infested potting mixture (from above). Thirty seeds which received no bacterial treatment are also planted in *Rhizoctonia*-infested pots. Twelve uninoculated seeds are planted in uninfested soil as a control sample aimed at detecting any potential for a component of the uninfested soil to cause disease symptoms.

(a3) Plant growth conditions: The pots are randomized within stainless steel trays and placed in a phytotron. Growth conditions are set at 20/23°C day/night with 14 hours of light per 24 hour period. Humidity in the phytotron is 70 %. Plants are watered on Day 7 by dispensing approximately 15 ml of sterilized distilled water into each pot.

(a4) Examination of cotton seedlings for evidence of *R. solani*-induced disease (Table 5): Cotton seedlings are removed from each pot at Day 12, and potting mixture is removed from the crown and root area by hand. The crown area (the region at and just below the soil line) is observed for evidence of brown lesions characteristic of post-emergence damping-off disease caused by *R. solani*. Healthy seedlings are scored as those showing no visual evidence of lesions.

Table 5: Percentage of healthy seedlings resulting from each treatment

| Treatment | % Healthy |
|---|---|
| No bacteria | 29 |
| *P.fluorescens* 900 (pLAFR3) | 38 |
| *P.fluorescens* 900 (pCIB3321) | 58 |
| *P.fluorescens* 900 (pCIP3306) | 71 |
| *P.fluorescens* 900 (pCIB3321) and *P.fluorescens* 900 (pCIP3306) | 57 |
| No bacteria, no *Rhizoctonia* | 100 |

(b) The conditions in (b) are identical to those in (a) above with the following exceptions:

(b1) The potting mixture is a 2: 1:1 mixture of commercial potting soil (Metromix 360):sand:vermiculite.

(b2) *R. solani*-infested millet seed is added at a rate of 15 mg/180 ml potting mixture (i.e. higher disease pressure than in (a).

(b3) *P.fluorescens* 900 (pCIP3306) is the only plant PR protein expresser tested.

(b4) In addition to bacterization by the seed soaking method of (a), 1 ml of the appropriate diluted bacterial culture is dispended on and around each bacterized seed prior to covering the seed with potting mixture. Results are presented in Table 6.

Table 6: Percentage of healthy seedlings resulting from each treatment

| Treatment | % Healthy |
|---|---|
| No bacteria | 19 |
| *P.fluorescens* 900 (pLAFR3) | 18 |
| *P.fluorescens* 900 (pCIP3306) | 44 |
| No bacteria, no *Rhizoctonia* | 100 |

(c) The conditions in (c) are identical to those of (b) with the following exception -*P.fluorescens* strain 924 containing pCIB3306 replaces *P.fluorescens* 900 (pCIP3306) in this experiment. Likewise, *P.fluorescens* 924 (pLAFR3) replaces *P.fluorescens* 900 (pLAFR3) as the negative control strain. Both pCI3306 and pLAFR3 are introduced by conjugation into the respective *P.fluorescens* 924 recipients using the triparental mating procedure (described in Example 1f). The results are presented in Table 7.

Table 7: Percentage of healthy seedlings resulting from each treatment

| Treatment | % Healthy |
|---|---|
| No bacteria | 21 |
| *P.fluorescens* 924 (pLAFR3) | 27 |
| *P.fluorescens* 924 (pCIP3306) | 56 |
| No bacteria, no *Rhizoctonia* | 97 |

While the present invention has been described with reference to specific embodiments thereof, it will be appreciated that numerous variations, modifications, and embodiments are possible, and accordingly, all such variations, modifications and embodiments are to be regarded as being within the spirit and scope of the present invention.

References

Better, M., Chang, C.P., Robinson, R.R., Horwitz, A.H., Science 240: 1041-1043 (1988)
Boller, T., UCLA Symposia on Molecular and Cellular Biology, New Series 22:247-262 (1985)
Burnette, W.N., Anal. Biochem. 112:195-203 (1981)
Carr and Klessig, Genetic Engineering, Principles and Methods 11:65-109, Plenum Press, New York ( 1989)
Crouse, G.F., Frischauf, A., Lehrach, H., Methods in Enzymology 101:78-89 (1983)
Davis, K.R., Ausubel, F.M., Molecular Plant-Microbe Interactions 2:363-368 (1989)
Deretic, V., Konyecsni, W.M., Mohr, C.D., Martin, D.W., Hibler, N.S., Bio/Technology 7: 1249- 1254 (1989)
Deuschle, U., Kammerer, W., Gentz, R., Bujard, H., EMBO J. 5:2987-2994 (1986)
Ditta, G., Stanfield, S., Corbin, D., Helinski, D.R., Proc. Natl. Acad. Sci. USA 77:7347-7351 ( 1980)
Gaynor, J.J., Nucleic Acids Res. 16:5210 (1988)

Gubler, U., Hoffman, B.J., Gene 25:263-269 ( 1983)
Hawley, D.K., McClure, W.R., Nucleic Acids Res. 11:2237-2255 (1983)
von Heijne, G., J. Mol. Biol. 184:99-105 (1985)
von Heijne, G., EMBO J. 3:2315-2318 (1984)
Horton, R.M., Hunt, H.D., Ho, S.N., Pullen, J.K., Pease, L.R., Gene 77:61-68 (1989)
Huang, J., Schell, M.A., J. Biol. Chem. 265: 11628- 11632 (1990)
Kombrik, E., Schröder, M., Hahlbrock, K., Proc. Natl. Acad. Sci. USA 85:782-786 (1985)
Maniatis, T., Fritsch, E.F., Sambrook, J., Molecular cloning, a laboratory manual, Cold Spring Harbor Laboratory, Cold Spring Harbor (1982)
Mauch, F., Mauch-Mani, B., Boller, T., Plant Phys. 88:936-942 (1988a)
Mauch, F., Hadwiger, L.A., Boller, T., Plant Physiol. 87:325-333 (1988b)
McCutcheon's Detergents and Emulsifiers Annual, MC Publishing Corp., Ringwood, New Jersey (1979)
Nasser, W., de Tapia, M., Kauffmann, S., Montasser-Kouhsari, S., Burkard, G., Plant Mol. Biol. 11:529-538 (1988)
Roberts, W.K., Selitrennikoff, C.P., J. Gen. Microbiol. 134: 169-176 (1988)
Rosenberg, M., Court, D., Ann. Rev. Genet. 13:319-353 (1979)
Saiki, R.K., Gelfand, D.H., Stoffel, S., Scharf, S.J., Higuchi, R., Horn, G.T., Mullis, K.B., Erlich, H.A., Science 239:487-491 (1988)
Shinshi, H., Mohnen, D., Meins, F., Proc. Natl. Acad. Sci. USA 84:89-93 (1987)
Shinshi, H., Wenzler, H., Neuhaus, J.-M., Felix, G., Hofsteenge, J., Meins, F., Proc. Natl. Acad. Sci. USA 85:5541-5545 (1988)
Sisely, Wood, Encyclopedia of Surface Active Agents, Chemical Publishing Co., New York (1980)
Sjöström, M., Wold, S., Wieslander, Å., Rilfors, L., EMBO J. 6:823-831 (1987)
Staskawicz, B., Dahlbeck, D., Keen, N., Napoli, C., J. Bacteriol. 169:5789-5794 (1987)
Vögeli, U., Meins, F., Boller, T., Planta 174:364-372 (1988)
Weller, D.M., Ann. Rev. Phytopathol. 26:379-407 ( 1988)

## Sequence listing

SEQ ID NO:1
SEQUENCE TYPE: Nucleotide
SEQUENCE LENGTH: 809 base pairs

```
ATTCAAGATA CAACATTTCT CCTATAGTCA TGGGATTTGT TCTCTTTTCA   50
CAATTGCCTT CATTTCTTCT TGTCTCTACA CTTCTCTTAT TCCTAGTAAT  100
ATCCCACTCT TGCCGTGCCC AAAATTCTCA ACAAGACTAT TTGGATGCCC  150
ATAACACAGC TCGTGCAGAT GTAGGTGTAG AACCTTTGAC CTGGGACGAC  200
CAGGTAGCAG CCTATGCGCA AAATTATGCT TCCCAATTGG CTGCAGATTG  250
TAACCTCGTA CATTCTCATG GTCAATACGG CGAAAACCTA GCTGAGGGAA  300
GTGGCGATTT CATGACGGCT GCTAAGGCTG TTGAGATGTG GGTCGATGAG  350
AAACAGTATT ATGACCATGA CTCAAATACT TGTGCACAAG GACAGGTGTG  400
TGGACACTAT ACTCAGGTGG TTTGGCGTAA CTCGGTTCGT GTTGGATGTG  450
CTAGGGTTCA GTGTAACAAT GGAGGATATG TTGTCTCTTG CAACTATGAT  500
CCTCCAGGTA ATTATAGAGG CGAAAGTCCA TACTAATTGA AACGACCTAC  550
GTCCATTTCA CGTTAATATG TATGGATTGT TCTGCTTGAT ATCAAGAACT  600
TAAATAATTG CTCTAAAAAG CAACTTAAAG TCAAGTATAT AGTAATAGTA  650
CTATATTTGT AATCCTCTGA AGTGGATCTA TAAAAAGACC AAGTGGTCAT  700
AATTAAGGGG AAAAATATGA GTTGATGATC AGCTTGATGT ATGATCTGAT  750
ATTATTATGA ACACTTTTGT ACTCATACGA ATCATGTGTT GATGGTCTAG  800
CTACTTGCG                                               809
```

SEQ ID NO:2
SEQUENCE TYPE: Nucleotide
SEQUENCE LENGTH: 771 base pairs

```
GTTCAAAATA AAACATTTCT CCTATAGTCA TGGGATTTTT TCTCTTTTCA   50
CAAATGCCCT CATTTTTTCT TGTCTCTACA CTTCTCTTAT TCCTAATAAT  100
ATCTCACTCT TCTCATGCCC AAAACTCTCA ACAAGACTAT TTGGATGCCC  150
ATAACACAGC TCGTGCAGAT GTAGGCGTGG AACCATTAAC TTGGGACAAC  200
GGGGTAGCAG CCTATGCACA AAATTATGTT TCTCAATTGG CTGCAGACTG  250
CAACCTCGTA CATTCTCATG GCCAATACGG CGAAAACCTA GCTCAGGGAA  300
GTGGCGATTT TATGACGGCT GCTAAGGCCG TCGAGATGTG GGTCGATGAG  350
AAACAGTACT ATGACCATGA CTCAAATACT TGTGCACAAG GACAGGTGTG  400
```

```
TGGACACTAT ACTCAGGTGG TTTGGCGTAA CTCGGTTCGT GTTGGATGTG   450
CTAGGGTTAA GTGCAACAAT GGAGGATATG TTGTCTCTTG CAACTATGAT   500
CCTCCAGGTA ATGTCATAGG CCAAAGTCCA TACTAATTGA AATGAATGTC   550
CATTTCACGT TATATATGTA TGGACTTCTG CTTGATATAT ATAAACAACT   600
TAAATAATTG CACTAAAAAG CAACTTATAG TTAAAAGTAT ATAATATTTG   650
TAATCCTCTG AAGAACTGGA TCTGTAAAAA GTCCAAGTGG TCTTAATTAA   700
GGGGGGGAGG ATATATGAAT TCAGCTTGAT GTATGATCTG ATATTATTAT   750
GAACTCTTTA GTACTCTTAC G                                  771
```

SEQ ID NO:3

SEQUENCE TYPE: Nucleotide

SEQUENCE LENGTH: 696 base pairs

```
GTTCAAAATA AAACATTTCT CCTATAGTCA TGGAATTTGT TCTCTTTTCA    50
CAAATGTCTT CATTTTTTCT TGTCTCTACG CTTCTCTTAT TCCTAATAAT   100
ATCCCACTCT TGTCATGCTC AAAACTCTCA ACAAGACTAT TTGGATGCCC   150
ATAACACAGC TCGTGCAGAT GTAGGTGTAG AACCTTTGAC CTGGGACGAC   200
CAGGTAGCAG CCTATGCACA AAATTATGCT TCCCAATTGG CTGCAGATTG   250
TAACCTCGTA CATTCTCATG GTCAATACGG CGAAAACCTA GCTTGGGGAA   300
GTGGCGATTT CTTGACGGCC GCTAAGGCCG TCGAGATGTG GGTCAATGAG   350
AAACAGTATT ATGCCCACGA CTCAAACACT TGTGCCCAAG GACAGGTGTG   400
TGGACACTAT ACTCAGGTGG TTTGGCGTAA CTCGGTTCGT GTTGGATGTG   450
CTAGGGTTCA GTGTAACAAT GGAGGATATA TTGTCTCTTG CAACTATGAT   500
CCTCCAGGTA ATGTTATAGG CAAAAGCCCA TACTAATTGA AAACATATGT   550
CCATTTCACG TTATATATGT GTGGACTTCT GCTTGATATA TATCAAGAAC   600
TTAAATAATT GCGCTAAAAA GCAACTTATA GTTAAGTATA TAGTACTATA   650
TTTGTAATTC TCTGAAGTGG ATATATAATA AGACCTAGTG CTCTTG       696
```

SEQ ID NO:4

SEQUENCE TYPE: Nucleotide

SEQUENCE LENGTH: 900 base pairs

```
AAAAAGAAAA AAAAAATGAA CTTCCTCAAA AGCTTCCCCT TTTTTGCCTT    50
CCTTTATTTT GGCCAATACT TTGTAGCTGT TACTCATGCT GCCACTTTTG   100
ACATTGTCAA CAAATGCACC TACACAGTCT GGGCCGCGGC CTCTCCAGGT   150
GGAGGCAGGC GGCTCGACTC AGGCCAATCT TGGAGCATTA ATGTGAACCC   200
```

AGGAACAGTC CAGGCTCGCA TTTGGGGTCG AACCAATTGC AACTTCGATG 250
GCAGTGGCCG AGGTAATTGT GAGACTGGAG ACTGTAACGG GATGTTAGAG 300
TGTCAAGGCT ATGGAAAAGC ACCTAACACT TTAGCTGAAT TTGCACTTAA 350
TCAACCCAAT CAGGACTTTG TCGACATCTC TCTTGTTGAT GGATTTAACA 400
TCCCCATGGA ATTCAGCCCG ACCAATGGAG GATGTCGTAA TCTCAGATGC 450
ACAGCACCTA TTAACGAACA ATGCCCAGCA CAGTTGAAAA CACAAGGTGG 500
ATGTAACAAC CCATGTACTG TGATAAAAAC CAATGAATAT TGTTGTACAA 550
ATGGGCCTGG ATCATGTGGG CCTACTGATT TGTCGAGATT TTTTAAGGAA 600
AGATGCCCTG ATGCTTATAG TTATCCACAG GATGATCCAA CCAGTTTGTT 650
TACGTGTCCT TCTGGTACTA ATTACAGGGT TGTCTTCTGC CCTTGAAATT 700
GAAGCCTGCA AAATTATGAC TATGTAATTT GTAGTTTCAA ATATATAAGC 750
TACACAAGTA GTACTAAGCA CTATTAAATA AAAAAGAGAG TGACAAAGAG 800
GAGAGGCTGT GGGTCAGATT CTCTTGTTCG CTGTTGTCGT TGTTGTAGCA 850
TTCTGGTTTT AAGAAATAAA GAAGATATAT ATCTGCTAAA TTATTAAATG 900

SEQ ID NO:5
SEQUENCE TYPE: Nucleotide
SEQUENCE LENGTH: 1020 base pairs

AAAAAAAAAA AAAAACATAA GAAAGTACAG AGGAAAATGG AGTTTTCTGG 50
ATCACCAATG GCATTGTTTT GTTGTGTGTT TTTCCTGTTC TTAACAGGGA 100
GCTTGGCACA AGGCATTGGT TCTATTGTAA CGAGTGACTT GTTCAACGAG 150
ATGCTGAAGA ATAGGAACGA CGGTAGATGT CCTGCCAATG GCTTCTACAC 200
TTATGATGCA TTCATAGCTG CTGCCAATTC CTTTCCTGGT TTTGGAACTA 250
CTGGTGATGA TACTGCCCGT AGGAAAGAAA TTGCTGCCTT TTTCGGTCAA 300
ACTTCTCACG AAACTACTGG TGGATCCCTG AGTGCAGAAC CATTTACAGG 350
AGGGTATTGC TTTGTTAGGC AAAATGACCA GAGTGACAGA TATTATGGTA 400
GAGGACCCAT CCAATTGACA AACCGAAATA ACTATGAGAA AGCTGGAACT 450
GCAATTGGAC AAGAGCTAGT TAACAACCCT GATTTAGTGG CCACAGATGC 500
TACTATATCA TTCAAAACAG CTATATGGTT TTGGATGACA CCACAGGACA 550
ACAAGCCATC TTCCCACGAC GTTATCATCG GTCGTTGGAC TCCGTCTGCC 600
GCGGATCAGG CGGCGAATCG AGTACCAGGT TACGGTGTAA TTACCAACAT 650
CATTAACGGT GGAATTGAAT GTGGCATAGG ACGGAATGAC GCAGTGGAAG 700
ATCGAATTGG ATACTACAGG AGGTATTGTG GTATGTTAAA TGTTGCTCCG 750
GGGGAAAACT TGGACTGTTA CAACCAAAGG AACTTCGGCC AGGGCTAGGC 800
TTCGTTACAT AGAATGCAGA TCATGTTATG TATACAAGTT ATATTTGTAT 850

TAATTAATGA ATAAGGGGAT TGTGTATCCA TTAAGAATTA GGTGAAATAT 900
TTCTGTTATT TGTCTTCTTG GGAAGAACCA ATAGCTCCTA TATATGAGGC 950
GCTTTTAAGT GATGAGGCTA CTGCATTGAT GAAAACGAAA TTTCTATCCA 1000
GAAATAAAAG TTCCTTGTCT 1020

SEQ ID NO:6
SEQUENCE TYPE: Nucleotide
SEQUENCE LENGTH: 978 base pairs

GAAAATGGAG TTTTCTGGAT CACCACTGAC ATTGTTTTGT TGTGTGTTTT 50
TCCTGTTCCT AACAGGGAGC TTGGCACAAG GCATTGGCTC AATTGTAACG 100
AATGACTTGT TCAACGAGAT GCTGAAGAAT AGGAACGACG GTAGATGTCC 150
TGCCAATGGC TTCTATACTT ATGATGCATT CATAGCTGCT GCCAATTCCT 200
TTCCTGGTTT TGGAACTAGT GGTGATGATA CTGCCCGTAG GAAAGAAATT 250
GCTGCCTTTT TCGGTCAAAC TTCTCATGAA ACTACAGGTG GTTCCCTGAG 300
TGCAGAACCT TTTACAGGAG GATATTGCTT TGTTAGGCAA AATGACCAGA 350
GTGACAGATA TTATGGTAGA GGACCCATCC AATTGACAAA CCAAAATAAC 400
TATGAGAAAG CTGGAAATGC AATTAGACAA GACCTAGTTA ACAACCCAGA 450
TTTAGTAGCT ACAGATGCTA CTATATCATT CAAAACAGCT ATATGGTTCT 500
GGATGACACC ACAGGATAAT AAGCCATCAA GCCACGACGT TATCATCGGT 550
AGTTGGACTC CGTCCGCCGC TGATCAGTCG GCGAATCGAG CACCTGGTTG 600
CGGTGTAATT ACCAACATTA TTAACGGTGG AATTGAATGT GGCGTAGGTC 650
CGAATGCCGC AGTGGAAGAT CGAATTGGAT ACTACAGGAG GTATTGTGGT 700
ATGTTGAATG TTGCTCCTGG GGACAACTTG GACTGTTACA ACCAAAGGAA 750
CTTCGCCCAA GGCTAGGATT CGTTAGATCA TGTTATGTGT ACACAAGTTA 800
TATTTGTATG TAATGAATAA GGGGATTGTG TACCCATTTA GAATAAGGGG 850
AAATATTTCT GTTATTTGTC TTCTTCGAAA GAATAACCAG TAGTTCCTAT 900
ATATCTGGTG CTTCGAGTGA AAACGAATAT TCTATCCGGA AATAAATACT 950
GTATGTTTCT TGTCTTAT 978

SEQ ID NO:7
SEQUENCE TYPE: Nucleotide
SEQUENCE LENGTH: 1108 base pairs

CAGGTGCTCA AGCAGGAGTT TGTTATGGAA GGCAAGGGAA TGGATTACCA 50
TCTCCAGCAG ATGTTGTGTC GCTATGCAAC CGAAACAACA TTCGTAGGAT 100

```
GAGAATATAT GATCCTGACC AGCCAACTCT CGAAGCGCTT AGAGGCTCCA   150
ACATTGAGCT CATGCTAGGT GTCCCGAATC CGGACCTTGA GAATGTTGCT   200
GCTAGCCAAG CCAATGCAGA TACTTGGGTC CAAAACAATG TTAGGAACTA   250
TGGTAATGTC AAGTTCAGGT ATATAGCAGT TGGAAATGAA GTTAGTCCCT   300
TAAATGAAAA CTCTAAGTAT GTACCTGTCC TTCTCAACGC CATGCGAAAC   350
ATTCAAACTG CCATATCTGG TGCTGGTCTT GGAAACCAGA TCAAAGTCTC   400
CACAGCTATT GAAACTGGAC TTACTACAGA CACTTCTCCT CCATCAAATG   450
GGAGATTCAA AGATGATGTT CGACAGTTTA TAGAGCCTAT CATCAACTTC   500
CTAGTGACCA ATCGCGCCCC TTTGCTTGTC AACCTTTATC CTTACTTTGC   550
AATAGCAAAC AATGCAGATA TTAAGCTTGA GTATGCACTT TTTACATCCT   600
CTGAAGTTGT TGTAAATGAT AACGGAAGAG GATACCGAAA CCTTTTTGAT   650
GCCATCTTAG ATGCCACATA CTCGGCCCTT GAAAAGGCTA GTGGCTCGTC   700
TTTGGAGATT GTTGTATCAG AGAGTGGTTG GCCTTCAGCT GGAGCAGGAC   750
AATTAACATC CATTGACAAT GCCAGGACTT ATAACAACAA CTTGATTAGT   800
CACGTGAAGG GAGGGAGTCC CAAAAGGCCT TCCGGTCCAA TAGAGACCTA   850
CGTTTTCGCT CTGTTTGATG AAGATCAGAA AGACCCTGAA ATTGAGAAGC   900
ATTTTGGACT ATTTTCAGCA AACATGCAAC CAAAGTACCA GATCAGTTTT   950
AACTAGTTAA AAGCAAGAGG AGAGCATTAA TAGGAATAAG GACTTTCCTT   1000
TGTATGAAGA GAAAGTAGTC CATTGGCACT ATGTACTGAA ACTATATATC   1050
ATGCTCATAA AGAAAGCAGT TATTACAATA ATGAAACACT TACAAGAAAA   1100
GCCATCAA                                                 1108
```

SEQ ID NO:8

SEQUENCE TYPE: Nucleotide

SEQUENCE LENGTH: 1195 base pairs

```
CTCAATTCTT GTTTTCCTTA CAAATGGCAC ATTTAATTGT CACACTGCTT   50
CTCCTTAGTG TACTTACATT AGCTACCCTG GATTTTACAG GTGCTCAAGC   100
AGGAGTTTGT TATGGAAGGC AAGGGAATGG ATTACCATCT CCAGCAGATG   150
TTGTGTCGCT ATGCAACCGA AACAACATTC GTAGGATGAG AATATATGAT   200
CCTGACCAGC CAACTCTCGA AGCGCTTAGA GGCTCCAACA TTGAGCTCAT   250
GCTAGGTGTC CCGAATCCGG ACCTTGAGAA TGTTGCTGCT AGCCAAGCCA   300
ATGCAGATAC TTGGGTCCAA AACAATGTTA GGAACTATGG TAATGTCAAG   350
TTCAGGTATA TAGCAGTTGG AAATGAAGTT AGTCCCTTAA ATGAAAACTC   400
TAAGTATGTA CCTGTCCTTC TCAACGCCAT GCGAAACATT CAAACTGCCA   450
TATCTGGTGC TGGTCTTGGA AACCAGATCA AAGTCTCCAC AGCTATTGAA   500
```

ACTGGACTTA CTACAGACAC TTCTCCTCCA TCAAATGGGA GATTCAAAGA 550
TGATGTTCGA CAGTTTATAG AGCCTATCAT CAACTTCCTA GTGACCAATC 600
GCGCCCCTTT GCTTGTCAAC CTTTATCCTT ACTTTGCAAT AGCAAACAAT 650
GCAGATATTA AGCTTGAGTA TGCACTTTTT ACATCCTCTG AAGTTGTTGT 700
AAATGATAAC GGAAGAGGAT ACCGAAACCT TTTTGATGCC ATCTTAGATG 750
CCACATACTC GGCCCTTGAA AAGGCTAGTG GCTCGTCTTT GGAGATTGTT 800
GTATCAGAGA GTGGTTGGCC TTCAGCTGGA GCAGGACAAT TAACATCCAT 850
TGACAATGCC AGGACTTATA ACAACAACTT GATTAGTCAC GTGAAGGGAG 900
GGAGTCCCAA AAGGCCTTCC GGTCCAATAG AGACCTACGT TTTCGCTCTG 950
TTTGATGAAG ATCAGAAAGA CCCTGAAATT GAGAAGCATT TTGGACTATT 1000
TTCAGCAAAC ATGCAACCAA AGTACCAGAT CAGTTTTAAC TAGTTAAAAG 1050
CAAGAGGAGA GCATTAATAG GAATAAGGAC TTTCCTTTGT ATGAAGAGAA 1100
AGTAGTCCAT TGGCACTATG TACTGAAACT ATATATCATG CTCATAAAGA 1150
AAGCAGTTAT TACAATAATG AAACACTTAC AAGAAAAGCC ATCAA 1195

SEQ ID NO:9
SEQUENCE TYPE: Nucleotide
SEQUENCE LENGTH: 1204 base pairs

GTGTTTCTTA CTCTCTCATT TCCATTTTAG CTATGACTTT ATGCATTAAA 50
AATGGCTTTC TTGCAGCTGC CCTTGTACTT GTTGGGCTGT TAATTTGCAG 100
TATCCAAATG ATAGGGGCAC AATCTATTGG AGTATGCTAT GGAAAACATG 150
CAAACAATTT ACCATCAGAC CAAGATGTTA TAAACCTATA CGATGCTAAT 200
GGCATCAGAA AGATGAGAAT CTACAATCCA GATACAAATG TCTTCAACGC 250
TCTCAGAGGA AGTAACATTG AGATCATTCT CGACGTCCCA CTTCAAGATC 300
TTCAATCCCT AACTGATCCT TCAAGAGCCA ATGGATGGGT CCAAGATAAC 350
ATAATAAATC ATTTCCCAGA TGTTAAATTT AAATATATAG CTGTTGGAAA 400
TGAAGTCTCT CCCGGAAATA ATGGTCAATA TGCACCATTT GTTGCTCCTG 450
CCATGCAAAA TGTATATAAT GCATTAGCAG CAGCAGGGTT GCAAGATCAA 500
ATCAAGGTCT CAACTGCAAC ATATTCAGGG ATCTTAGCGA ATACCAACCC 550
GCCCAAAGAT AGTATTTTTC GAGGAGAATT CAATAGTTTC ATTAATCCCA 600
TAATCCAATT TCTAGTACAA CATAACCTTC CACTCTTAGC CAATGTCTAT 650
CCTTATTTTG GTCACATTTT CAACACTGCT GATGTCCCAC TTTCTTATGC 700
TTTGTTCACA CAACAAGAAG CAAATCCTGC AGGATATCAA AATCTTTTTG 750
ATGCCCTTTT GGATTCTATG TATTTTGCTG TAGAGAAAGC TGGAGGACAA 800
AATGTGGAGA TTATTGTATC TGAAAGTGGC TGGCCTTCTG AAGGAAACTC 850

TGCAGCAACT ATTGAAAATG CTCAAACTTA CTATGAAAAT TTGATTAATC 900
ATGTGAAAAG CGGGGCAGGA ACTCCAAAGA AACCTGGAAA TGCTATAGAA 950
ACTTATTTAT TTGCCATGTT TGATGAAAAT AATAAGGAAG GAGATATCAC 1000
AGAGAAACAC TTTGGACTCT TTTCTCCTGA TCAGAGGGCA AAATATCAAC 1050
TCAATTTCAA TTAATTAATG CATGGTAACA TTTATTGATA TATATAGTGA 1100
TATGAGTAAT AAGGAGAAGT AGAACTGCTA TGTTTTTCTC TTCAATTGAA 1150
AATGTAACTC TGGTTTCACT TTGATATTTA TATGACATGT TTATTGAGAT 1200
CTAA 1204

SEQ ID NO:10
SEQUENCE TYPE: Nucleotide
SEQUENCE LENGTH: 529 base pairs

ACAGTAAAAA ACTGAAACTC CAAATAGCTC ATCAAAATGT TTTCCAAAAC 50
TAACCTTTTT CTTTGCCTTT CTTTGGCTAT TTTGCTAATT GTAATATCCT 100
CACAAGCTGA TGCAAGGGAG ATGTCTAAGG CGGCTGTTCC AATTACCCAA 150
GCAATGAATT CAAACAACAT TACTAATCAG AAGACGGGTG CCGGAATCAT 200
CCGTAAGATA CCGGGTTGGA TACGAAAAGG TGCAAAACCA GGAGGCAAAG 250
TCGCCGGCAA AGCTTGTAAA ATTTGCTCAT GTAAATACCA GATTTGCAGC 300
AAATGTCCTA AATGTCATGA CTAAAGTTAG GCCTTGAGAC TATGTACTTG 350
TGCTGGTGTG AGTTTAGTTT TGAGAGTAAA GGGAAAGTTA TGAATAGCCT 400
AATATAATTG TATTCACTAT GTTTTCTTAG TAATTCTTAT TGTTGAAACT 450
TGGAACAGGT CTTTGGGTCA AAATGTACCT CTTGTCTTGT AGTCTTTCAA 500
CTGTATAGTA TTGTACTGTA TTTTTCTTT 529

SEQ ID NO:11
SEQUENCE TYPE: Nucleotide
SEQUENCE LENGTH: 560 base pairs

GACAGTAAAA AACTGAAACT CCAAATAGCT CATCAAAATG TTTTCCAAAA 50
CTAACCTTTT TCTTTGCCTT TCTTTGGCTA TTTTGCTAAT TGTAATATCC 100
TCACAAGCTG ATGCAAGGCA GATTTCTAAG GCGGCTGCTC CAATTACCCA 150
TGCAATGAAT TCAAACAACA TTACTAATCA GAAGACGGGT GCCGGAATCA 200
TCCGTAAGAT ACCGGGTTGG ATACGAAAAG GTGCAAAACC AGGAGGCAAA 250
GTCGCCGGCA AAGCTTGTAA AATTTGCTCA TGTAAATACC AGATTTGCAG 300
CAAATGTCCT AAATGTCATG ACTAAAGTTA GGCCTTGAGA CTATGTACTT 350

GTGCTGGTGT GAGTTTAATT TTGAGAGTAA AGGGAAAGTT ATGAATAGCC 400
TAATATAATT CTATTCACTA TGTTTTCTTA GTAATTCTTA TTGTTGAAAC 450
TTGGAACAGG TCTTTGGGTC AAAATGTACC TCTTGTCTTG TAGTCTTTCA 500
ACTGTATGGT ATTGTACTGT ATCTTTCTTT AGCCACTTGA TATCAAATCC 550
GATTAAATCT 560

SEQ ID NO:12
SEQUENCE TYPE: Nucleotide
SEQUENCE LENGTH: 1103 base pairs

AAAGAAAGCT CTTTAAGCAA TGGCTGCCCA CAAAATAACT ACAACCCTTT 50
CCATCTTCTT CCTCCTTTCC TCTATTTTCC GCTCTTCCGA CGCGGCTGGA 100
ATCGCCATCT ATTGGGGTCA AAACGGCAAC GAGGGCTCTC TTGCATCCAC 150
CTGCGCAACT GGAAACTACG AGTTCGTCAA CATAGCATTT CTCTCATCCT 200
TTGGCAGCGG TCAAGCTCCA GTTCTCAACC TTGCTGGTCA CTGCAACCCT 250
GACAACAACG GTTGCGCTTT TTTGAGCGAC GAAATAAACT CTTGCAAAAG 300
TCAAAATGTC AAGGTCCTCC TCTCTATCGG TGGTGGCGCG GGGAGTTATT 350
CACTCTCCTC CGCCGACGAT GCGAAACAAG TCGCAAACTT CATTTGGAAC 400
AGCTACCTTG GCGGGCAGTC GGATTCCAGG CCACTTGGCG CTGCGGTTTT 450
GGATGGCGTT GATTTCGATA TCGAGTCTGG CTCGGGCCAG TTCTGGGACG 500
TACTAGCTCA GGAGCTAAAG AATTTTGGAC AAGTCATTTT ATCTGCCGCG 550
CCGCAGTGTC CAATACCAGA CGCTCACCTA GACGCCGCGA TCAAAACTGG 600
ACTGTTCGAT TCCGTTTGGG TTCAATTCTA CAACAACCCG CCATGCATGT 650
TTGCAGATAA CGCGGACAAT CTCCTGAGTT CATGGAATCA GTGGACGGCG 700
TTTCCGACAT CGAAGCTTTA CATGGGATTG CCAGCGGCAC GGGAGGCAGC 750
GCCGAGCGGG GGATTTATTC CGGCGGATGT GCTTATTTCT CAAGTTCTTC 800
CAACCATTAA AGCTTCTTCC AACTATGGAG GAGTGATGTT ATGGAGTAAG 850
GCGTTTGACA ATGGCTACAG CGATTCCATT AAAGGCAGCA TCGGCTGAAG 900
GAAGCTCCTA AGTTTAATTT TAATTAAAGC TATGAATAAA CTCCAAAGTA 950
TTATAATAAT TAAAAAGTGA GACTTCATCT TCTCCATTTA GTCTCATATT 1000
AAATTAGTGT GATGCAATAA TTAATATCCT TTTTTTCATT ACTATACTAC 1050
CAATGTTTTA GAATTGAAAA GTTGATGTCA ATAAAAACAT TCCAAGTTTA 1100
TTT 1103

SEQ ID NO:13
SEQUENCE TYPE: Nucleotide
SEQUENCE LENGTH: 1131 base pairs

```
ACCAGAGAAG ACCCCATTTG CAGTATCAAA AATGGGTTTA CCTAAAATGG   50
CAGCCATTGT TGTGGTGGTG GCTTTGATGC TATCACCCTC TCAAGCCCAG  100
YTTTCTCCTT TCTTCTACGC CACCACATGC CCTCAGCTGC CTTTCGTTGT  150
TCTCAACGTG GTTGCCCAAG CCCTACAGAC TGATGACCGA GCTGCTGCTA  200
AGCTCATTCG CCTCCATTTT CATGATTGCT TTGTCAATGG GTGTGATGGA  250
TCGATTCTAT TGGTAGACGT ACCGGGCGTT ATCGATAGTG AACTTAATGG  300
ACCTCCAAAT GGTGGAATCC AAGGAATGGA CATTGTGGAC AACATCAAAG  350
CAGCAGTTGA GAGTGCTTGT CCAGGAGTTG TTTCTTGCGC TGATATCTTA  400
GCCATTTCAT CTCAAATCTC TGTTTTCTTG TCGGGAGGAC CAATTTGGGT  450
TGTACCAATG GGAAGAAAAG ACAGCAGAAT AGCCAATAGA ACTGGAACCT  500
CAAACTTACC TGGTCCCTCA GAAACTCTAG TGGGACTTAA AGGCAAGTTT  550
AAAGATCAAG GGCTTGATTC TACAGATCTC GTGGCTCTAT CAGGAGCCCA  600
CACGTTTGGA AAATCAAGAT GCATGTTCTT CAGTGACCGC CTCATCAACT  650
TCAACGGCAC AGGAAGACCC GACACAACGC TTGACCCAAT ATACAGGGAG  700
CAGCTTCGAA GACTTTGTAC TACTCAACAA ACACGAGTAA ATTTCGACCC  750
AGTCACACCC ACTAGATTTG ACAAGACCTA TTACAACAAT TTGATTAGCT  800
TAAGAGGGCT TCTCCAAAGC GACCAAGAGC TCTTCTCAAC TCCCAGAGCT  850
GATACCACAG CCATTGTCAR AACTTTTGCT GCCAACGAAC GTGCCTTCTT  900
TAAACAATTT GTGAAATCAA TGATCAAAAT GGGCAACCTC AAGCCTCCCC  950
CTGGCATTGC ATCAGAAGTT AGATTGGACT GTAAGAGGGT CAACCCAGTC 1000
AGAGCCTACG ACGTTATGTA ATAACTTTAT CCCACTTCAT CCCTTCTACT 1050
TTTGCTGTCT CTTGTACTAC TTTGTTGATG TATTAGTTCA ACCGGTTAAG 1100
ATATATATAT CGTTGACCTA AATAATAGAT C                     1131
```

SEQ ID NO:14
SEQUENCE TYPE: Nucleotide with corresponding protein
SEQUENCE LENGTH: 133 base pairs

```
TTTAAACACT CAAGTTAATC AAGGATCTGA TTACTC ATG ACA AAC CCC   48
                                        Met Thr Asn Pro

TCA ACC TTC ACT GCC TCC AAA CTG GCC AGT GCC GTT ATC GGT  100
Ser Thr Phe Thr Ala Ser Lys Leu Ala Ser Ala Val Ile Gly

GCC CTG CTG TTG TCC AGC GTT CCT GCG CAT GCT              133
Ala Leu Leu Leu Ser Ser Val Pro Ala His Ala
```

SEQ ID NO:15
SEQUENCE TYPE: Nucleotide
SEQUENCE LENGTH: 25 bases

AGCTCTTTAA GCC**ATG**GCTG CCCAC 25

SEQ ID NO:16
SEQUENCE TYPE: Nucleotide
SEQUENCE LENGTH: 15 bases

CTACGAGTTG CATGA 15

SEQ ID NO:17
SEQUENCE TYPE: Nucleotide
SEQUENCE LENGTH: 30 bases

TTTATGTGAG GTAGACATGG TCTGTTTCCT 30

SEQ ID NO:18
SEQUENCE TYPE: Nucleotide
SEQUENCE LENGTH: 30 bases

GGAAACAGAC CATGTCTACC TCACATAAAC 30

SEQ ID NO:19

SEQUENCE TYPE: Nucleotide

SEQUENCE LENGTH: 16 bases

```
TGCATGTTCC ATTCCG                                          16
```

SEQ ID NO:20

SEQUENCE TYPE: Nucleotide with corresponding protein

SEQUENCE LENGTH: 1358 base pairs

```
CCTCAAA TGG CTG CTA TCA CAC TCC TAG GAT TAC TAC TTG TTG   43
        Met Ala Ala Ile Thr Leu Leu Gly Leu Leu Leu Val

CCA GCA GCA TTG ACA TAG CAG GGG CTC AAT CGA TAG GTG TTT   85
Ala Ser Ser Ile Asp Ile Ala Gly Ala Gln Ser Ile Gly Val

GCT ATG GAA TGC TAG GCA ACA ACT TGC CAA ATC ATT GGG AAG  127
Cys Tyr Gly Met Leu Gly Asn Asn Leu Pro Asn His Trp Glu

TTA TAC AGC TCT ACA AGT CAA GAA ACA TAG GAA GAC TGA GGC  169
Val Ile Gln Leu Tyr Lys Ser Arg Asn Ile Gly Arg Leu Arg

TTT ATG ATC CAA ATC ATG GAG CTT TAC AAG CAT TAA AAG GCT  211
Leu Tyr Asp Pro Asn His Gly Ala Leu Gln Ala Leu Lys Gly

CAA ATA TTG AAG TTA TGT TAG GAC TTC CCA ATT CAG ATG TGA  253
Ser Asn Ile Glu Val Met Leu Gly Leu Pro Asn Ser Asp Val

AGC ACA TTG CTT CCG GAA TGG AAC ATG CAA GAT GGT GGG TAC  295
Lys His Ile Ala Ser Gly Met Glu His Ala Arg Trp Trp Val

AGA AAA ATG TTA AAG ATT TCT GGC CAG ATG TTA AGA TTA AGT  337
Gln Lys Asn Val Lys Asp Phe Trp Pro Asp Val Lys Ile Lys

ATA TTG CTG TTG GGA ATG AAA TCA GCC CTG TCA CTG GCA CAT  379
Tyr Ile Ala Val Gly Asn Glu Ile Ser Pro Val Thr Gly Thr

CTT ACC TAA CCT CAT TTC TTA CTC CTG CTA TGG TAA ATA TTT  421
Ser Tyr Leu Thr Ser Phe Leu Thr Pro Ala Met Val Asn Ile

ACA AAG CAA TTG GTG AAG CTG GTT TGG GAA ACA ACA TCA AGG  463
Tyr Lys Ala Ile Gly Glu Ala Gly Leu Gly Asn Asn Ile Lys

TCT CAA CTT CTG TAG ACA TGA CCT TGA TTG GAA ACT CTT ATC  505
Val Ser Thr Ser Val Asp Met Thr Leu Ile Gly Asn Ser Tyr

CAC CAT CAC AGG GTT CGT TTA GGA ACG ATG CTA GGT GGT TTA  547
Pro Pro Ser Gln Gly Ser Phe Arg Asn Asp Ala Arg Trp Phe
```

CTG ATC CAA TTG TTG GGT TCT TAA GGG ACA CAC GTG CAC CTT 589
Thr Asp Pro Ile Val Gly Phe Leu Arg Asp Thr Arg Ala Pro

TAC TCG TTA ACA TTT ACC CCT ATT TCA GCT ATT CTG GTA ATC 631
Leu Leu Val Asn Ile Tyr Pro Tyr Phe Ser Tyr Ser Gly Asn

CAG GGC AGA TTT CTC TCC CCT ATT CTC TTT TTA CAG CAC CAA 673
Pro Gly Gln Ile Ser Leu Pro Tyr Ser Leu Phe Thr Ala Pro

ATG TGG TAG TAC AAG ATG GTT CAC GCC AAT ATA GGA ACT TAT 715
Asn Val Val Val Gln Asp Gly Ser Arg Gln Tyr Arg Asn Leu

TTG ATG CAA TGC TGG ATT CTG TGT ATG CTG CCC TCG AGC GAT 757
Phe Asp Ala Met Leu Asp Ser Val Tyr Ala Ala Leu Glu Arg

CAG GAG GGG CAT CTG TAG GGA TTG TTG TGT CCG AGA GTG GCT 799
Ser Gly Gly Ala Ser Val Gly Ile Val Val Ser Glu Ser Gly

GGC CAT CTG CTG GTG CAT TTG GAG CCA CAT ATG ACA ATG CAG 841
Trp Pro Ser Ala Gly Ala Phe Gly Ala Thr Tyr Asp Asn Ala

CAA CTT ACT TGA GGA ACT TAA TTC AAC ACG CTA AAG AGG GTA 883
Ala Thr Tyr Leu Arg Asn Leu Ile Gln His Ala Lys Glu Gly

GCC CAA GAA AGC CTG GAC CTA TTG AGA CCT ATA TAT TTG CCA 925
Ser Pro Arg Lys Pro Gly Pro Ile Glu Thr Tyr Ile Phe Ala

TGT TTG ATG AGA ACA ACA AGA ACC CTG AAC TGG AGA AAC ATT 967
Met Phe Asp Glu Asn Asn Lys Asn Pro Glu Leu Glu Lys His

TTG GAT TGT TTT CCC CCA ACA AGC AGC CCA AAT ATA ATA TCA 1009
Phe Gly Leu Phe Ser Pro Asn Lys Gln Pro Lys Tyr Asn Ile

ACT TTG GGG TCT CTG GTG GAG TTT GGG ACA GTT CAG TTG AAA 1051
Asn Phe Gly Val Ser Gly Gly Val Trp Asp Ser Ser Val Glu

CTA ATG CTA CTG CTT CTC TCG TAA GTG AGA TGT GAGCTGATGAG 1095
Thr Asn Ala Thr Ala Ser Leu Val Ser Glu Met

ACACTTGAAA TCTCTTTACA TACGTATTCC TTGGATGGAA AACCTAGTAA 1145
AAACAAGAGA AATTTTTTCT TTATGCAAGA TACTAAATAA CATTGCATGT 1195
CTCTGTAAGT CCTCATGGAT TGTTATCCAG TGACGATGCA ACTCTGAGTG 1245
GTTTTAAATT CCTTTTCTTT GTGATATTGG TAATTTGGCA AGAAACTTTC 1295
TGTAAGTTTG TGAATTTCAT GCATCATTAA TTATACATCA GTTCCATGTT 1345
TGATCAAAAA AAA 1358

SEQ ID NO:21
SEQUENCE TYPE: Nucleotide
SEQUENCE LENGTH: 28 bases

CATCTGAATT CTCCCAACAA GTCTTCCC 28

SEQ ID NO:22
SEQUENCE TYPE: Nucleotide
SEQUENCE LENGTH: 42 bases

AACACCTATC GATTGAGCCC CTGCTATGTC AATGCTGGTG GC 42

SEQ ID NO:23
SEQUENCE TYPE: Nucleotide
SEQUENCE LENGTH: 81 base pairs

GGATCCGTTT GCATTTCACC AGTTTACTAC TACATTAAAA TGAGGCTTTG 50
TAAATTCACA GCTCTCTCTT CTCTACTATT T 81

SEQ ID NO:24
SEQUENCE TYPE: Nucleotide
SEQUENCE LENGTH: 31 bases

CAAAACTCTC AACAAGACTA TTTGGATGCC C 31

SEQ ID NO:25
SEQUENCE TYPE: Nucleotide
SEQUENCE LENGTH: 16 bases

GAGTGAGCTG ATACCG 16

SEQ ID NO:26
SEQUENCE TYPE: Nucleotide
SEQUENCE LENGTH: 30 bases

ACACCTATCG ATTGAGCATG CGCAGGAACG 30

SEQ ID NO:27
SEQUENCE TYPE: Nucleotide
SEQUENCE LENGTH: 179 base pairs

FEATURES:
from 69 to 73 predicted ribosome binding site
from 85 coding region beginning with the ATG start codon of the pectate lyase leader peptide
at 171/172 fusion junction between the pectate lyase leader peptide sequence and the coding region of the mature portion of the tobacco basic β-1,3-glucanase
sequence begins with the *Asp*718 site (GGTACC) and ends with the *Cla*I site (ATCGAT).

```
GGTACCCGGG GATCCTCTAG AGTCGAGAAA CGCGCATGCC TGTGCGCGTT   50
TAAACACTCA AGTTAATCAA GGATCTGATT ACTCATGACA AACCCCTCAA  100
CCTTCACTGC CTCCAAACTG GCCAGTGCCG TTATCGGTGC CCTGCTGTTG  150
TCCAGCGTTC CTGCGCATGC TCAATCGAT                         179
```

SEQ ID NO:28
SEQUENCE TYPE: Nucleotide with corresponding protein
SEQUENCE LENGTH: 96 base pairs

```
ATG TCT ACC TCA CAT AAA CAT AAT ACT CCT CAA ATG GCT GCT 42
Met Ser Thr Ser His Lys His Asn Thr Pro Gln Met Ala Ala

ATC ACA CTC CTA GGA TTA CTA CTT GTT GCC AGC AGC ATT GAC 84
Ile Thr Leu Leu Gly Leu Leu Leu Val Ala Ser Ser Ile Asp

ACA GCA GGG GCT                                         96
Thr Ala Gly Ala
```

SEQ ID NO:29
SEQUENCE TYPE: Nucleotide
SEQUENCE LENGTH: 16 bases

```
GAGTGAGCTG ATACCG                                       16
```

SEQ ID NO:30
SEQUENCE TYPE: Nucleotide
SEQUENCE LENGTH: 41 bases

```
GACCCCAATA GATGGCGATT CCAGCAGCAT GCGCAGGAAC G                41
```

## Claims

1. A DNA sequence comprising:
   a) regulatory components from a bacterial source fused to
   b) a DNA coding sequence from a plant, which DNA coding sequence results in expression of a plant pathogenesis related protein in a bacterium.

2. A DNA sequence according to claim 1 wherein said bacterium is a soil bacterium.

3. A DNA sequence according to claim 1 wherein said plant pathogenesis related protein is a glycosidase.

4. A DNA sequence according to claim 3 wherein said glycosidase is selected from the group consisting of chitinases and glucanases.

5. A DNA sequence according to claim 3 wherein said glycosidase is selected from the group consisting of PR-1A, PR-1B, PR-1C, PR-R major, PR-R minor, PR-P, PR-Q, PR-Q′, PR-2, PR-N, PR-O, PR-O′, cucumber chitinase/lysozyme, tobacco basic glucanase and tobacco basic chitinase.

6. A DNA sequence according to claim 1 wherein said plant pathogenesis related protein is selected from the group consisting of SAR8.2a, SAR8.2b and cucumber basic peroxidase.

7. A recombinant bacterium containing one or more DNA sequences according to claim 1.

8. A method for providing plant protective activity in a localized environment comprising introducing into the localized environment one or more bacterial strains, wherein each of the bacterial strains contains a DNA sequence of claim 1, said DNA sequence being capable of producing a plant protective amount of a pathogenesis related protein.

9. The method according to claim 8 wherein said localized environment is a rhizosphere.

10. The method according to claim 8 comprising introducing into a localized environment a mixture of more than one bacterial strain, with the proviso that each bacterial strain in the mixture contains a different DNA sequence, said different DNA sequences being capable of producing different pathogenesis related proteins in the same localized environment resulting in an enhanced plant protective activity.

11. The method according to claim 10 wherein said localized environment is a rhizosphere.

12. A method for providing plant protective activity in a localized environment comprising introducing into the localized environment a bacterial strain containing more than one of the DNA sequences of claim 1, each of said DNA sequences being capable of producing a plant protective effective amount of a pathogenesis related protein.

13. The method according to claim 12 wherein said localized environment is a rhizosphere.

## Claims for the following Contracting State : ES

1. A method for providing plant protective activity in a localized environment comprising introducing into the localized environment one or more bacterial strains, wherein each of the bacterial strains contains a DNA sequence comprising a) regulatory components from a bacterial source fused to b) a DNA coding sequence from a plant, said DNA sequence being capable of producing a plant protective amount of a pathogenesis related protein.

2. The method according to claim 1 wherein said bacterial strain is a soil bacterium.

3. The method according to claim 1 wherein said plant pathogenesis related protein is a glycosidase.

4. The method according to claim 3 wherein said glycosidase is selected from the group consisting of chitinases and glucanases.

5. The method according to claim 3 wherein said glycosidase is selected from the group consisting of PR-1A, PR-1B, PR-1C, PR-R major, PR-R minor, PR-P, PR-Q, PR-Q′, PR-2, PR-N, PR-O, PR-O′, cucumber chitinase/lysozyme, tobacco basic glucanase and tobacco basic chitinase.

6. The method according to claim 1 wherein said plant pathogenesis related protein is selected from the group consisting of SAR8.2a, SAR8.2b and cucumber basic peroxidase.

7. The method according to claim 1 wherein said localized environment is a rhizosphere.

8. The method according to claim 1 comprising introducing into a localized environment a mixture of more than one bacterial strain, with the proviso that each bacterial strain in the mixture contains a different DNA sequence, said different DNA sequences being capable of producing different pathogenesis related proteins in the same localized environment resulting in an enhanced plant protective activity.

9. The method according to claim 8 wherein said localized environment is a rhizosphere.

10. A method for providing plant protective activity in a localized environment comprising introducing into the localized environment a bacterial strain containing more than one of DNA sequences comprising a) regulatory components from a bacterial source fused to b) a DNA coding sequence from a plant, each of said DNA sequences being capable of producing a plant protective effective amount of a pathogenesis related protein.

11. The method according to claim 10 wherein said bacterial strain is a soil bacterium.

12. The method according to claim 10 wherein said plant pathogenesis related protein is a glycosidase.

13. The method according to claim 12 wherein said glycosidase is selected from the group consisting of chitinases and glucanases.

14. The method according to claim 12 wherein said glycosidase is selected from the group consisting of PR-1A, PR-1B, PR-1C, PR-R major, PR-R minor, PR-P, PR-Q, PR-Q′, PR-2, PR-N, PR-O, PR-O′, cucumber chitinase/lysozyme, tobacco basic glucanase and tobacco basic chitinase.

15. The method according to claim 10 wherein said plant pathogenesis related protein is selected from the group consisting of SAR8.2a, SAR8.2b and cucumber basic peroxidase.

16. The method according to claim 10 wherein said localized environment is a rhizosphere.